# EUROPEAN PATENT SPECIFICATION

(11) **EP 1 534 275 B1**
(45) Date of publication and mention of the grant of the patent: **12.03.2008**
(21) Application number: 03784054.3
(22) Date of filing: 24.07.2003
(51) Int. Cl.: A61K 31/4184, C07D 235/30, C07D 401/06, C07D 413/12, C07D 403/12, C07D 417/12, C07D 409/12, C07D 401/12, C07D 277/82, A61P 9/00

(54) **ACYLAMINO-SUBSTITUTED HETEROAROMATIC COMPOUNDS AND THEIR USE AS PHARMACEUTICALS**
ACYLAMINO-SUBSTITUIERTE HETEROAROMATISCHE VERBINDUNGEN UND IHRE VERWENDUNG ALS ARZNEIMITTEL
COMPOSES HETEROAROMATIQUES ACYLAMINO-SUBSTITUES ET LEUR UTILISATION EN TANT QUE PRODUITS PHARMACEUTIQUES

(30) Priority: 07.08.2002 EP 02017585
(43) Date of publication of application: 01.06.2005
(73) Proprietor: Sanofi-Aventis Deutschland GmbH, 65929 Frankfurt am Main (DE)
(72) Inventor: STROBEL, Hartmut, 65835 Liederbach (DE); WOHLFART, Paulus, 64625 Bensheim (DE); BELOW, Peter, 60529 Frankfurt (DE)
(86) International application number: PCT/EP2003/008102
(87) International publication number: WO 2004/014369

(56) References cited:
- WO-A-00/27819
- WO-A-01/83427
- WO-A-02/34711
- WO-A-02/064146
- DATABASE ZCAPLUS [Online] KHRISTICH, B.I.; ET AL.: "Benzimidazole derivatives. XXXII.Synthesis and transformations of 2-amino-3-acyl-1-methylbenzimidazolium" retrieved from STN, accession no. 1975:43256 Database accession no. 82:43256 XP002217425 & KHIM.GETEROTSIKL.SOEDIN., vol. 10, 1974, pages 1398-1401,
- DATABASE CROSSFIRE BEILSTEIN [Online] Beilstein Institut zur Förderung der Chemischen Wissenschaften, Frankfurt am Main, DE; Database accession no. 923029 XP002217426 & CHEM.HETEROCYCL.COMD. (ENG. TRANSL.), vol. 10, 1974, pages 1225-1227,
- DATABASE CROSSFIRE BEILSTEIN [Online] Beilstein Institut zur Förderung der Chemischen Wissenschaften, Frankfurt am Main, DE; Database accession no. 837542 XP002217427 & J. GEN. CHEM USSR (ENGL. TRANSL.), vol. 32, 1962, page 2194
- DATABASE CROSSFIRE BEILSTEIN [Online] Beilstein Institut zur Förderung der Chemischen Wissenschaften, Frankfurt am Main, DE; Database accession no. 925285 XP002217428 & CHEM.HETEROCYCL.COMPD. (ENG.TRANSL.), vol. 10, 1974, pages 1225-1227,
- DATABASE CROSSFIRE BEILSTEIN [Online] Beilstein Institut zur Förderung der Chemischen Wissenschaften, Frankfurt am Main, DE; Database accession no. 617400 XP002217429 & J.HETEROCYCL.CHEM., vol. 25, 1988, pages 931-935,
- DATABASE CROSSFIRE BEILSTEIN [Online] Beilstein Institut zur Förderung der Chemischen Wissenschaften, Frankfurt am Main, DE; Database accession no. 5965541 XP002217430 & SYNTH.COMMUN., vol. 18, no. 16-17, 1988, pages 1995-2002,
- DATABASE CROSSFIRE BEILSTEIN [Online] Beilstein Institut zur Förderung der Chemischen Wissenschaften, Frankfurt am Main, DE; Database accession no. 5973047 XP002217431 & SYNTH.COMMUN., vol. 18, no. 16-17, 1988, pages 1995-2002,
- DATABASE CROSSFIRE BEILSTEIN [Online] Beilstein Institut zur Förderung der Chemischen Wissenschaften, Frankfurt am Main, DE; Database accession no. 5978659 XP002217432 & SYNTH.COMMUN:, vol. 18, no. 16-17, 1988, pages 1995-2002,
- DATABASE CROSSFIRE BEILSTEIN [Online] Beilstein Institut zur Förderung der Chemischen Wissenschaften, Frankfurt am Main, DE; Database accession no. 3558042 XP002217433 & CHEM.HETEROCYCL.COMPD. (ENGL.TRANSL), vol. 25, no. 2, 1989, page 229
- LAUFS U ET AL: "UPREGULATION OF ENDOTHELIAL NITRIC OXIDE SYNTHASE BY HMG COA REDUCTASE INHIBITORS" CIRCULATION, AMERICAN HEART ASSOCIATION, DALLAS, TX, US, vol. 97, no. 12, 1998, pages 1129-1135, XP000870148 ISSN: 0009-7322
- DATABASE CROSSFIRE BEILSTEIN [Online] Beilstein Institut zur Förderung der Chemischen Wissenschaften, Frankfurt am Main, DE; Database accession no. 31710 XP002217434 & ZH.OBSHCH.KHIM., vol. 14, 1944, page 315
- DATABASE CROSSFIRE BEILSTEIN [Online] Beilstein Institut zur Förderung der Chemischen Wissenschaften, Frankfurt am Main, DE; Database accession no. 748697 XP002217435 & CHEM.BER., vol. 105, 1972, pages 813-819,
- DATABASE CROSSFIRE BEILSTEIN [Online] Beilstein Institut zur Förderung der Chemischen Wissenschaften, Frankfurt am Main, DE; Database accession no. 763346 XP002217436 & J.HETR.CHEM., vol. 16, 1979, pages 321-324,
- DATABASE CROSSFIRE BEILSTEIN [Online] Beilstein Institut zur Förderung der Chemischen Wissenschaften, Frankfurt am Main, DE; Database accession no. 235421 XP002217437 & J.AMER.CHEM.SOC., vol. 70, 1948, page 2572

## Description

The present invention relates to acylamino-substituted heteroaromatic compounds of the formula la, 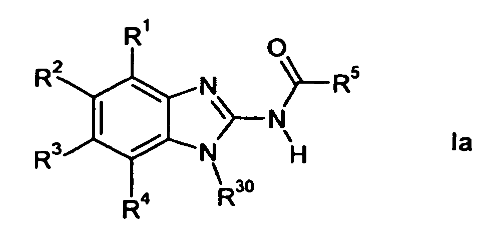 in which R¹, R², R³, R⁴, R⁵ and R³⁰ have the meanings indicated below. The compounds of formula la are valuable pharmaceutically active compounds which are useful in the treatment of various disease states including cardiovascular disorders such as atherosclerosis, thrombosis, coronary artery disease, hypertension and cardiac insufficiency. They upregulate the expression of the enzyme endothelial nitric oxide (NO) synthase and can be applied in conditions in which an increased expression of said enzyme or an increased NO level or the normalization of a decreased NO level is desired. The invention furthermore relates to processes for the preparation of compounds of the formula la, their use, in particular as active ingredients in pharmaceuticals, and pharmaceutical preparations comprising them.

Endothelial NO synthase (eNOS, NOS-III) belongs to a group of three isoenzymes which produce nitric oxide (NO) by oxidation of arginine. Endothelially released NO is of central importance in a number of key cardiovascular mechanisms. It has a vasodilating effect and inhibits the aggregation of platelets, the adhesion of leukocytes to the endothelium and the proliferation of intimal smooth muscle cells.

Endothelial NO synthase is subject to physiological and pathophysiological regulation both at the transcriptional and at the post-transcriptional level. Enzyme already present in the endothelium may undergo calcium-dependent and calcium-independent activation through phosphorylation of specific amino acids, but also by direct interactions with specific proteins. Stimulators of this, usually transient, NO release are, extracellular arginine, 17β-estrogen and the mechanical stimulus exerted on the luminal surface of the endothelium by the blood flow (shear stress). The latter additionally leads to regulation of eNOS at the transcriptional level. Thus, for example, Sessa et al. (Circ. Research 74 (1994) 349) were able by means of exercise training and the increase in shear stress associated therewith to obtain a marked increase in eNOS.

Whether regulation at the post-transcriptional level is relevant in vivo, has not been unambiguously proven. Thus, for example, administration of a high arginine dose is followed by only a transient improvement in the endothelium-dependent vasorelaxation in patients with coronary heart disease.

On the other hand, the significance of the upregulation of the eNOS protein is scientifically accepted. Thus, there are findings which show that the protective properties of the. HMG-CoA reductase inhibitor simvastatin can be attributed, besides to the lipid lowering effect, also in part to an increase in eNOS expression in vivo (Endres et al., Proc. Natl. Acad. Sci. USA 95 (1998) 8880; cf. Laufs et al., Circulation 97 (1998) 1129). It is additionally known that single point mutations in the 5'-flanking region of the eNOS gene ("eNOS promoter"), and the reduction in the rate of eNOS gene transcription associated therewith, in the Japanese population is associated with an increase in the risk of coronary spasms (Nakayama et al., Circulation 99 (1999) 2864).

The current assumption therefore is that the transcriptional and post-transcriptional mechanisms of eNOS regulation are seriously disturbed in a large number of disorders, especially in cardiovascular disorders. Even in very early stages of a wide variety of cardiovascular disorders it is possible for a dysfunction of this type in the endothelium lining the blood vessels to lead to a deficiency of bioactive NO, which is manifested as the disorder progresses in the form of measurable pathophysiological and morphological changes. Thus, critical steps in early atherogenesis are speeded up by a decrease in endothelial NO release, such as, for example, the oxidation of low density lipoproteins, the recruitment and deposition of monocytes in the intima of vessels, and the proliferation of intimal cells. A consequence of atherogenesis is the formation of plaques on the inside of the blood vessels, which may in tum lead, through a diminution in the shear stress, to a further decrease in endothelial NO release and a further deterioration in the pathology. Since endothelial NO is also a vasodilator, a decrease thereof frequently also leads to hypertension, which may, as an independent risk factor, cause further organ damage.

The aim of a therapeutic approach to the treatment of these disorders must accordingly be to interrupt this chain of events by increasing the endothelial NO expression. Gene transfer experiments which lead in vitro to overexpression of NO synthase in previously damaged vessels are in fact able to counteract the described processes and are thus evidence of the correctness of this approach (Varenne et al., Hum. Gene Ther. 11 (2000) 1329).

Some low molecular weight compounds which, in cell cultures, may lead to a direct effect on eNOS transcription and expression are disclosed in the literature. The statins which have already been mentioned are, however, the only substances for which it has been possible to date to show such an increase in eNOS in vivo as a side effect. But in view of the known range of side effects of this class of substances it is unclear how far this effect is present in a toxicologically unproblematic dose.

Liao et al. claim in WO 99/47153 and WO 00/03746 the use of rhoGTPase inhibitors and agents which influence the organization of the actin cytoskeleton for increasing eNOS in endothelial cells and for the therapy of various disorders such as, for example, stroke or pulmonary hypertension without, however, indicating a specific way of achieving this.

WO 02/064146, WO 02/064545, WO 02/064565 and WO 02/064546 disclose acylated, benzo-condensed cycloalkenylamines which upregulate eNOS expression in endothelial cells and are useful pharmaceutically active ingredients for the treatment of various diseases, but there is an ongoing need for further eNOS expression enhancers with a favorable property profile. The present invention satisfies this need by providing compounds of the formula Ia and methods of using them.

Certain acylamino-substituted heteroaromatic compounds of the formula Ia and structurally similar compounds have already been described. In many cases, the known compounds have been prepared in the course of merely chemical investigations or for use as intermediates in the synthesis of other compounds, and no biological activity of them has been described. Compounds of the formula la in which R¹, R², R³ and R⁴ are all hydrogen and R⁵ is 4-nitrophenyl, 3,4-methylenedioxyphenyl, 4-bromophenyl, 4-methoxyphenyl, unsubstituted phenyl or 2-chlorophenyl have been described in Khim. Geterotsikl. Soedin. (1974), 1398 (Chem. Heterocycl. Compd. (Engl. Transl.) 10 (1974) 1225); J. Heterocycl. Chem. 25 (1988) 931; and Chem. Heterocycl. Compd. (Engl. Transl.) 25 (1989) 229. Compounds of the formula la in which R¹, R³ and R⁴ are all hydrogen, R² is chloro and R⁵ is 4-methyl phenyl, 4-methoxyphenyl or unsubstituted phenyl have been described in Synth. Commun. 18 (1988) 1995. The compound N-(1,5-dimethyl-1H-benzimidazol-2-yl)-4-nitrobenzamide has been described in J. Gen. Chem. USSR (Engl. Transl.) 32 (1962) 2194. Compounds of the formula la and structurally similar compounds for which a pharmaceutical activity has already been described include, for example, certain hypotensive 2-acetylaminobenzimidazoles (Bellasio et al., Farmaco, Ed. Sci., 28 (1973) 164) which, however, do not suggest the 2-(hetero)aroylamino-benzimidazoles comprised by the present invention and their biological activity. The activating effect on guanylate cyclase of certain N-benzimidazolylcarboxamides and N-benzothiazolylcarboxamides described in WO 00/27394 seems to depend on the presence of a 3-(3-(dimethylamino)propoxy)-1-(phenylmethyl)-1H-pyrazol-5-carboxamide moiety. In WO 01/97786 the adenosine receptor affinity of certain N-benzothiazolylcarboxamides has been disclosed. In WO 01/83427, WO 98/11073 and Pilyugin et al., Bashkirskii Khimicheskii Zhurnal 8 (2001) 18, it has been described that similar compounds exhibit a hypoglycemic activity, antiviral activity or antifungal activity, respectively. More specifically, in WO 01/83427 (EP 1277729) it is described that the comprised N-substituted 2-chloro-5-nitrobenzamides, among which is the compound 2-chloro-N-(1-methyl-1H-benzimidazol-2-yl)-5-nitrobenzamide, have PPAR y modulating activity. Among the compounds described in WO 02/034711, which inhibit trypsin-like serine protease enzymes such as thrombin, factor Vlla and factor Xa, for example, are 2-(4-isobutylcarbamoyl-2-methoxycarbonylphenyl)-N-(1-methyl-1 H-benzimidazol-2-yl)-benzamide and 2-(2-carboxy-4-isobutylcarbamoylphenyl)-N-(1-methyl-1H-benzimidazol-2-yl)-benzamide. Among the compounds described in WO 00/27819, which inhibit the VEGF receptor and angiogenesis, is the compound N-(1-methyl-1H-benzimidazol-2-yl)-2-((pyridin-4-ylmethyl)amino)benzamide. There are no indications in the prior art that compounds of the formula la would upregulate the expression of endothelial NO synthase, but it is surprising that they exhibit this activity and therefore are valuable pharmaceutically active ingredients which are useful in the treatment of a variety of diseases including cardiovascular diseases such as, for example, atherosclerosis, thrombosis, coronary artery disease, hypertension and cardiac insufficiency.

A subject of the present invention are novel acylamino-substituted heteroaromatic compounds of the formula la, 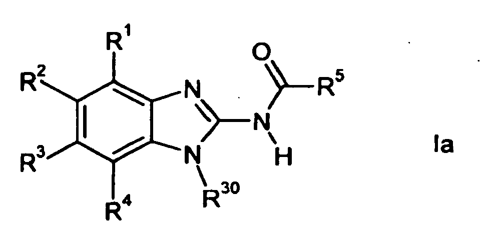 in any of their stereoisomeric forms and mixtures thereof in any ratio, and the pharmaceutically acceptable salts thereof, wherein in the formula la:
R¹ and R⁴ are independently from each other selected from the group consisting of: H; unsubstituted and at least monosubstituted C₁-C₁₀-alkyl, C₂-C₁₀-alkenyl and C₂-C₁₀-alkynyl, the substituents of which are selected from the group consisting of F, OH, C₁-C₈-alkoxy, C₁-C₈-alkylmercapto, -CN, COOR⁶, CONR⁷R⁸, and unsubstituted and at least monosubstituted phenyl and heteroaryl where the substituents of the phenyl and heteroaryl groups are selected from the group consisting of halogens, -CN, C₁-C₃-alkyl, C₁-C₃-alkoxy and CF₃; unsubstituted and at least monosubstituted phenyl and heteroaryl the substituents of which are selected from the group consisting of halogens, -CN, C₁-C₃-alkyl, C₁-C₃-alkoxy and CF₃; COR⁹; CONR¹⁰R¹¹; COOR¹²; CF₃; halogens; -CN; NR¹³R¹⁴; OR¹⁵; S(O)ₘR¹⁶; SO₂NR¹⁷R¹⁸; and NO₂;
R² and R³ are independently from each other selected from the group consisting of: H; halogens; -CN; unsubstituted and at least monosubstituted C₁-C₁₀-alkyl the substituents of which are selected from the group consisting of OH, phenyl, and heteroaryl; OH; C₁-C₁₀-alkoxy; phenoxy; S(O)ₘR¹⁹; CF_{3;} -CN; NO₂; C₁-C₁₀-alkylamino; di(C₁-C₁₀-alkyl)amino; (C₁-C₆-alkyl)-CONH-; unsubstituted and at least monosubstituted phenyl-CONH- and phenyl-SO₂-O- the substituents of which are selected from the group consisting of halogens, -CN, methyl and methoxy; C₁-C₆-alkyl-SO₂-O-; unsubstituted and at least monosubstituted (C₁-C₆-alkyl)-CO- the substituents of which are selected from the group consisting of F, di(C₁-C₃-alkyl)amino, pyrrolidinyl and piperidinyl; and phenyl-CO- the phenyl part of which is unsubstituted or at least monosubstituted by substituents selected from the group consisting of C₁-C₃-alkyl, halogens and methoxy;
R⁵ is a group Ar which is unsubstituted or carries one or more identical or different substituents selected from the group consisting of:
   halogens; -CN; NH₂; unsubstituted and at least monosubstituted C₁-C₁₀-alkyl, C₂-C₁₀-alkenyl, C₂-C₁₀-alkynyl, C₁-C₁₀alkoxy, C₁-C₁₀-alkylamino and di(C₁-C₁₀-alkyl)amino, the substituents of which are selected from the group consisting of F, OH, C₁-C₈-alkoxy, aryloxy, C₁-C₈-alkylmercapto, NH₂, C₁-C₈-alkylamino and di(C₁-C₈-alkyl)amino; C₃-C₅-alkandiyl; phenyl; heteroaryl; aryl-substituted or heteroaryl-substituted C₁-C₄-alkyl; CF₃; OH; phenoxy; benzyloxy; (C₁-C₁₀-alkyl)-COO-; S(O)rnR²⁰; SH; phenylamino; benzylamino; (C₁-C₁₀-alkyl)-CONH-; (C₁-C₁₀-alkyl)-CON(C₁-C₄-alkyl)-; phenyl-CONH-; phenyl-CO-N(C₁-C₄-alkyl)-; heteroaryl-CONH-; heteroaryl-CO-N(C₁-C₄-alkyl)-; (C₁-C₁₀-alkyl)-CO-;-phenyl-CO-; heteroaryl-CO-; CF₃-CO-; -OCH₂O-; -OCF₂O-; -OCH₂CH₂O-; -CH₂CH₂O-; COOR²¹; CONR²²R²³; C(NH)-NH₂; SO₂NR²⁴R²⁵; R²⁶SO₂NH-; R²⁷SO₂N(C₁-C₆-alkyl)-; and a residue of a saturated or at least monounsaturated aliphatic, monocyclic 5-membered to 7-membered heterocycle containing 1, 2 or 3 heteroatoms selected from the group consisting of N, O and S, which heterocycle can be substituted by one or more substituents selected from the group consisting of halogens, C₁-C₃-alkyl, C₁-C₃-alkoxy, OH, oxo and CF₃, where said heterocycle can optionally be condensed to the said group Ar; wherein all aryl, heteroaryl, phenyl, aryl-containing, heteroaryl-containing and phenyl-containing groups, which are optionally present in the said substituents of the said group Ar, can be substituted by one or more substituents selected from the group consisting of halogens, -CN, C₁-C₃-alkyl, OH, C₁-C₃-alkoxy, and CF₃;
R⁶ is selected from the group consisting of:
   H; C₁-C₁₀-alkyl which can be substituted by one or more substituents selected from the group consisting of F, C₁-C₈-alkoxy and di(C₁-C₈-alkyl)amino; aryl-(C₁-C₄-alkyl)-and heteroaryl-(C₁-C₄-alkyl)- both of which can be substituted by one or more substituents selected from the group consisting of halogens, C₁-C₄-alkyl, C₁-C₄-alkoxy and di(C₁-C₆-alkyl)amino;
R⁷ is selected from the group consisting of:
   H; C₁-C₁₀-alkyl which can be substituted by one or more substituents selected from the group consisting of F, C₁-C₈-alkoxy, di(C₁-C₈-alkyl)amino and phenyl; phenyl; indanyl; and heteroaryl; wherein each of the aromatic groups can be unsubstituted or carry one or more substituents selected from the group consisting of halogens, -CN, C₁-C₃-alkyl, C₁-C₃-alkoxy and CF₃;
R⁸ is H or C₁-C₁₀-alkyl;
R⁹ is selected from the group consisting of:
   C₁-C₁₀-alkyl which can be substituted by one or more substituents from the group consisting of F, C₁-C₄-alkoxy and di(C₁-C₃-alkyl)amino; and unsubstituted and at least monosubstituted phenyl and heteroaryl the substituents of which are selected from the group consisting of C₁-C₃-alkyl, C₁-C₃-alkoxy, halogens, -CN and CF₃;
R¹⁰, independently from R⁷, is defined as R⁷;
R¹¹, independently from R⁸, is defined as R⁸;
R¹², independently from R⁶, is defined as R⁶;
R¹³ is selected from the group consisting of:
   H; C₁-C₆-alkyl; unsubstituted and substituted phenyl, benzyl, heteroaryl, (C₁-C₆-alkyl)-CO-, phenyl-CO-, and heteroaryl-CO-, the substituents of which are selected from the group consisting of halogens, -CN, C₁-C₃-alkyl, C₁-C₃-alkoxy and CF₃, wherein one or more of these substituents can be present;
R¹⁴, independently from R¹³, is defined as R¹³;
R¹⁵ is selected from the group consisting of:
   H; C₁-C₁₀-alkyl; (C₁-C₃-alkoxy)-C₁-C₃-alkyl- and substituted and unsubstituted benzyl, phenyl and heteroaryl, the substituents of which are selected from the group consisting of halogens, -CN, C₁-C₃-alkyl, C₁-C₃-alkoxy and CF₃, wherein one or more of these substituents can be present;
R¹⁶ is selected from the group consisting of:
   C₁-C₁₀-alkyl which can be substituted by one or more substituents selected from the group consisting of F, OH, C₁-C₈-alkoxy, aryloxy, C₁-C₈-alkylmercapto, C₁-C₈-alkylamino and di(C₁-C₈-alkyl)amino; CF₃; and substituted and unsubstituted phenyl and heteroaryl, the substituents of which are selected from the group consisting of halogens, -CN, C₁-C₃-alkyl, C₁-C₃-alkoxy and CF₃, wherein one or more of these substitutents can be present;
R¹⁷, independently from R⁷, is defined as R⁷;
R¹⁸, independently from R⁸, is defined as R⁸;
R¹⁹, independently from R¹⁶, is defined as R¹⁶;
R²⁰, independently from R¹⁶, is defined as R¹⁶;
R²¹, independently from R⁶, is defined as R⁶;
R²², independently from R⁷, is defined as R⁷;
R²³, independently from R⁸, is defined as R⁸;
R²⁴, independently from R⁷, is defined as R⁷;
R²⁵, independently from R⁸, is defined as R⁸;
R²⁶, independently from R¹⁶, is defined as R¹⁶;
R²⁷, independently from R¹⁶, is defined as R¹⁶;
R³⁰ is methyl;
heteroaryl is a residue of a 5-membered to 10-membered, aromatic, monocyclic or bicyclic heterocycle containing one or more heteroatoms selected from the group consisting of N, O and S;
aryl is phenyl, naphth-1-yl or naphth-2-yl;
the group Ar is phenyl, naphth-1-yl or naphth-2-yl;
m is 0, 1 or 2;
provided that R⁵ cannot be phenyl, 2-chlorophenyl, 4-bromophenyl, 4-methoxyphenyl or 3,4-methylenedioxyphenyl if simultaneously R¹, R², R³ and R⁴ are all hydrogen; and that R⁵ cannot be phenyl, 4-methylphenyl or 4-methoxyphenyl if simultaneously R¹, R³ and R⁴ are all hydrogen and R² is chloro.

If groups or substituents in the compounds of the formula la such as, for example, aryl, heteroaryl, alkyl etc., can be present several times, they all independently from each other have the meanings indicated and can hence, in each individual case, be identical with or different from each other. As an example the di(C₁-C₁₀-alkyl)amino group may be mentioned in which the alkyl substitutents can be identical or different. When a group in the compounds of the formula la can be at least monosubstituted, or when it carries one or more substituents, it can be substituted, for example, by one, two, three, four or five substituents. When a group is substituted by two or more substituents, the substituents can be identical or different from each other.

Alkyl, alkenyl and alkynyl residues can be linear or branched, acyclic or cyclic. This also applies when they are part of other groups, for example alkoxy groups, alkoxycarbonyl groups or substituted amino groups, or when they are substituted.

Examples of alkyl groups are methyl, ethyl, propyl, butyl, pentyl, hexyl, heptyl, octyl, nonyl, decyl, the n-isomers of these residues, isopropyl, isobutyl, isopentyl, sec-butyl, tert-butyl, neopentyl, 3,3-dimethylbutyl. The term alkyl here also expressly includes cycloalkyl groups and cycloalkyl-alkyl- groups (i. e., alkyl substituted by cycloalkyl) which groups contain at least three carbon atoms. Examples of such cycloalkyl residues are cyclopropyl, cyclobutyl, cyclopentyl, cyclohexyl, cycloheptyl and cyclooctyl. All cycloalkyl groups can be substituted by one or more identical or different C₁-C₄-alkyl residues, in particular by methyl. Examples of substituted cycloalkyl residues are 4-methylcyclohexyl, 4-tert-butylcyclohexyl or 2,3-dimethylcyclopentyl. Furthermore, unless stated otherwise, the term alkyl here also includes unsubstituted alkyl residues as well as alkyl residues which are substituted by one or more, for example 1, 2, 3 or 4, identical or different residues, for example aryl groups. In substituted alkyl residues, for example arylalkyl-, hydroxyalkyl- such as hydroxy-(C₁-C₃)-alkyl- or alkoxyalkyl- such as C₁-C₄-alkyl-O-(C₁-C₃)-alkyl-, the substituents can be present in any desired position.

Examples of alkenyl and alkynyl groups are vinyl, 1-propenyl, 2-propenyl (i.e. allyl), 2-butenyl, 2-methyl-2-propenyl, 3-methyl-2-butenyl, ethynyl, 2-propynyl (i.e. propargyl), 2-butynyl or 3-butynyl. The term alkenyl here also expressly includes cycloalkenyl groups and cycloalkenyl-alkyl- groups (i.e. alkyl substituted by cycloalkenyl) which groups contain at least three carbon atoms. Examples of cycloalkenyl residues are cyclopentenyl, cyclohexenyl, cycloheptenyl and cyclooctenyl. All cycloalkenyl groups can be substituted by one or more identical or different C₁-C₄-alkyl residues, in particular by methyl. Furthermore, unless stated otherwise, the term alkenyl and alkynyl here also includes unsubstituted alkenyl and alkynyl, residues as well as alkenyl and alkynyl residues which are substituted by one or more, for example 1, 2, 3 or 4, identical or different residues, for example aryl groups. In substituted alkenyl and alkynyl residues, for example arylalkenyl-, hydroxyalkenyl- such as hydroxy-(C₂-C₃)-alkenyl- or alkoxyalkenyl- such as C₁-C₃-alkyl-O-(C₂-C₄-alkenyl)-, the substituents can be present in any desired position.

Examples of C₃-C₅-alkandiyl are -CH₂CH₂CH₂-, -CH₂-CH(CH₃)-, -CH₂CH₂CH₂CH₂- and -CH₂CH₂CH₂CH₂CH₂- groups.

If not stated otherwise, the above-mentioned phenyl residues, naphthyl and indanyl residues and heterocyclic residues (including heteroaryl residues) can be unsubstituted or can carry one or more, for example 1, 2, 3 or 4, of the substituents indicated in the above definition which substituents can be present in any desired position. If in compounds of the formula la nitro groups are present as substituents, in a preferred embodiment of the invention in total only up to two nitro groups are present in the molecule. In monosubstituted phenyl residues the substituent can be in the 2-position, the 3-position or the 4-position, in disubstituted phenyl residues the substituents can be in 2,3-position, 2,4-position, 2,5-position, 2,6-position, 3,4-position or 3,5-position. In trisubstituted phenyl residues the substituents can be in 2,3,4-position, 2,3,5-position, 2,3,6-position, 2,4,5-position, 2,4,6-position or 3,4,5-position. In fourfold substituted phenyl residues, the substituents can be in the 2,3,4,5-position, the 2,3,4,6-position, or the 2,3,5,6-position. Tolyl (= methylphenyl) can be 2-tolyl, 3-tolyl or 4-tolyl. Naphthyl can be 1-naphthyl or 2-naphthyl. In monosubstituted 1-naphthyl residues the substituent can be in the 2-position, the 3-position, the 4-position, the 5-position, the 6-position, the 7-position or the 8-position, in monosubstituted 2-naphthyl residues in the 1-position, the 3-position, the 4-position, the 5-position, the 6-position, the 7-position or the 8-position. In higher substituted naphthyl residues, for example 1-naphthyl residues or 2-naphthyl residues which carry two or three substituents, the substituents can be present in any desired positions. Indanyl residues include indan-1-yl residues and indan-2-yl residues which can be unsubstituted or carry one or more of the substituents indicated. In case the indanyl residues are substituted, the substituent or substituents can be present in any of the positions possible.

Unless stated otherwise, heteroaryl residues and heterocyclic residues are preferably derived from heterocycles which contain 1, 2, 3 or 4 heteroatoms which can be identical or different; more preferably they are derived from heterocycles which contain 1, 2 or 3, in particular 1 or 2, heteroatoms which can be identical or different. Unless stated otherwise, the heterocycles can be monocyclic or polycyclic, for example monocyclic, bicyclic or tricyclic. Preferably they are monocyclic or bicyclic. The number of ring members preferably is 5, 6, 8, 9 or 10. The individual rings preferably are 5-membered rings, 6-membered rings or 7-membered rings. Examples of monocyclic and bicyclic heterocyclic systems from which residues occurring in the compounds of the formula Ia can be derived, are pyrrole, furan, thiophene, imidazole, pyrazole, 1,2,3-triazole, 1,2,4-triazole, 1,3-dioxole, 1,3-oxazole (= oxazole), 1,2-oxazole (= isoxazole), 1,3-thiazole (= thiazole), 1,2-thiazole (= isothiazole), tetrazole, pyridine, pyridazine, pyrimidine, pyrazine, pyran, thiopyran, 1,4-dioxine, 1,2-oxazine, 1,3-oxazine, 1,4-oxazine, 1,2-thiazine, 1,3-thiazine, 1,4-thiazine, 1,2,3-triazine, 1,2,4-triazine, 1,3,5-triazine, 1,2,4,5-tetrazine, azepine, 1,2-diazepine, 1,3-diazepine, 1,4-diazepine, 1,3-oxazepine, 1,3-thiazepine, indole, benzothiophene, benzofuran, benzothiazole, benzoxazole, benzimidazole, benzodioxole, quinoline, isoquinoline, cinnoline, quinazoline, quinoxaline, phthalazine, thienothiophenes, 1,8-naphthyridine and other naphthyridines, pteridin, or phenothiazine, each of them in saturated form (perhydro form) or in partially unsaturated form (for example In the dihydro form or the tetrahydro form) or in maximally unsaturated form or aromatic form, provided that the respective forms are known and stable. The term "aryl" and the term "heteroaryl" as used herein comprise bicyclic residues in which both cycles are aromatic as well as bicyclic residues in which only one cycle is aromatic. The same applies to the term "group Ar". Suitable heterocycles include, for example, the saturated heterocycles pyrrolidine, piperidine, piperazine, morpholine and thiomorpholine. The degree of saturation of heterocyclic groups is indicated in their individual definitions. Unsaturated heterocycles can contain, for example, 1, 2 or 3, double bonds within the ring system. 5-membered rings and 6-membered rings can in particular also be aromatic.

Residues derived from the mentioned heterocycles can be attached via any suitable carbon atom. Residues derived from nitrogen heterocycles which can carry a hydrogen atom or a substituent on a ring nitrogen atom, such as pyrrole, imidazole, pyrrolidine, morpholine or piperazine residues, can also be attached via a ring nitrogen atom, in particular if the respective heterocyclic residue is attached to a carbon atom. For example, a thienyl residue can be present as 2-thienyl residue or 3-thienyl residue, a furyl residue as 2-furyl residue or 3-furyl residue, a pyridinyl residue as 2-pyridinyl residue, 3-pyridinyl residue or 4-pyridinyl residue, a piperidinyl residue as 1-piperidinyl residue (= piperidino residue), 2-piperidinyl residue, 3-piperidinyl residue or 4-piperidinyl residue, a (thio)morpholinyl residue as 2-(thio)morpholinyl residue, 3-(thio)morpholinyl residue or 4-(thio)morpholinyl residue (= thiomorpholino residue). A residue derived from 1,3-thiazole or imidazole which is attached via a carbon atom can be attached via the 2-position, the 4-position or the 5-position.

In case a heterocyclic groups is substituted, it can carry one or more, for example 1, 2, 3 or 4, identical or different substituents. Substituents in heterocycles can be present in any desired positions, for example in a 2-thienyl residue or 2-furyl residue in the 3-position and/or in the 4-position and/or in the 5-position, in a 3-thienyl residue or 3-furyl residue in the 2-position and/or in the 4-position and/or in the 5-position, in a 2-pyridinyl residue in the 3-position and/or in the 4-position and/or in the 5-position and/or in the 6-position, in a 3-pyridinyl residue in the 2-position and/or in the 4-position and/or in the 5-position and/or in the 6-position, in a 4-pyridinyl residue in the 2-position and/or in the 3-position and/or in the 5-position and/or in the 6-position. Suitable nitrogen heterocycles can also be present as N-oxides or as quarternary salts containing a counterion which is derived from a pharmaceutically acceptable acid. Pyridine moieties, for example, can thus be present as pyridine-N-oxides.

Halogen is fluorine, chlorine, bromine or iodine, preferably fluorine or chlorine.

The present invention includes all stereoisomeric forms of the compounds of the formula la. Centers of asymmetry that are present in the compounds of formula la all independently from one another can have S configuration or R configuration. The invention includes all possible enantiomers and diastereomers and mixtures of two or more stereoisomers, for example mixtures of enantiomers and/or diastereomers, in all ratios. Thus, compounds of the present invention which can exist as enantiomers can be present in enantiomerically pure form, both as levorotatory and as dextrorotatory antipodes, in the form of racemates and in the form of mixtures of the two enantiomers in all ratios. In the case of a cis/trans isomerism the invention includes both the cis form and the trans form as well as mixtures of these forms in all ratios. All these forms are a subject of the present invention. The preparation of individual stereoisomers can be carried out, if desired, by separation of a mixture by customary methods, for example by chromatography or crystallization, by the use of stereochemically uniform starting materials for the synthesis or by stereoselective synthesis. Optionally a derivatization can be carried out before a separation of stereoisomers. The separation of a mixture of stereoisomers can be carried out at the stage of the compounds of the formula Ia or at the stage of an intermediate during the synthesis or at the stage of a starting compound. The present invention also includes all tautomeric forms of the compounds of formula Ia.

In case the compounds of the formula Ia contain one or more acidic or basic groups, the invention also comprises their corresponding pharmaceutically or toxicologically acceptable salts, in particular their pharmaceutically utilizable salts. Thus, the compounds of the formula la which contain acidic groups can be present on these groups and can be used according to the invention, for example, as alkali metal salts, alkaline earth metal salts or as ammonium salts. Examples of such salts include sodium salts, potassium salts, calcium salts, magnesium salts or salts with ammonia or organic amines such as, for example, ethylamine, ethanolamine, triethanolamine or amino acids. Compounds of the formula la which contain one or more basic groups, i.e. groups which can be protonated, can be present and can be used according to the invention in the form of their addition salts with inorganic or organic acids. Examples of suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfamic acid, phenylpropionic acid, gluconic acid, ascorbic acid, isonicotinic acid, citric acid, adipic acid, and other acids known to the person skilled in the art. If the compounds of the formula Ia simultaneously contain acidic and basic groups in the molecule, the invention also includes, in addition to the salt forms mentioned, inner salts or betaines (zwitterions). The salts of the compounds of the formula Ia can be obtained by customary methods which are known to the person skilled in the art like, for example, by contacting the compound of the formula Ia with an organic or inorganic acid or base in a solvent or diluent, or by anion exchange or cation exchange from another salts. The present invention also includes all salts of the compounds of the formula Ia which, owing to low physiological compatibility, are not directly suitable for use in pharmaceuticals but which can be used, for example, as intermediates for chemical reactions or for the preparation of pharmaceutically acceptable salts.

The compounds of formula Ia can form solvates for example hydrates and adducts with alcohols, active metabolites, and also derivatives and prodrugs which contain physiologically tolerable and cleavable groups, for example esters, amides and compounds in which the N-H group depicted in formula la is replaced with an N-alkyl group, such as N-methyl, or with an N-acyl group, such as N-acetyl or N-argininyl, including pharmaceutically acceptable salts formed on functional groups present in the N-acyl group.

In preferred embodiments of the present invention, one or more of the structural moieties in the compounds of formula la, including the groups R¹, R², R³, R⁴, R⁵, R³⁰ and the other groups present in the compounds of formula Ia, independently from each other have the following preferred meanings, more preferred meanings, even more preferred meanings or most preferred meanings.

R¹ is preferably selected from the group consisting of: H; C₁-C₄-alkyl; C₁-C₄-alkoxy; CF₃; halogens; -CN; C₁-C₄-alkyl-S(O)ₘ-; and unsubstituted and at least monosubstituted phenyl and heteroaryl the substituents of which are selected from the group consisting of halogens, -CN, C₁-C₃-alkyl, C₁-C₃-alkoxy and CF₃, where heteroaryl is selected from the group consisting of 5-membered and 6-membered heterocycles containing one or more heteroatoms selected from the group consisting of N, O, and S. More preferably R¹ is H, halogen or C₁-C₄-alkyl.

R² is preferably selected from the group consisting of H, halogens, -CN and C₁-C₄-alkyl, more preferably from the group consisting of H, halogens and C₁-C₄-alkyl. Even more preferably R² is H.

R³ is preferably selected from the group consisting of H, halogens, -CN and C₁-C₄-alkyl, more preferably from the group consisting of H, halogens and C₁-C₄-alkyl. Even more preferably R³ is H.

R⁴ is preferably selected from the group consisting of: H; C₁-C₄-alkyl; C₁-C₄-alkoxy; CF₃; halogens; -CN; C₁-C₄-alkyl-S(O)ₘ-; and unsubstituted and at least monosubstituted phenyl and heteroaryl the substituents of which are selected from the group consisting of halogens, -CN, C₁-C₃-alkyl, C₁-C₃-alkoxy and CF₃, where heteroaryl is selected from the group consisting of 5-membered and 6-membered heterocycles containing one or more heteroatoms selected from the group consisting of N, O, and S. More preferably R⁴ is H, halogen or C₁-C₄-alkyl. Most preferably R⁴ is H.

In a preferred embodiment of the present invention R¹, R², R³ and R⁴ are all H. In another preferred embodiment of the invention at least one of the groups R¹, R², R³ and R⁴, for example one or two of them, is different from hydrogen.

R⁵ is preferably a group Ar which is unsubstituted or carries
one or more identical or different substituents selected from the group consisting of: halogens; -CN; NH₂; unsubstituted and at least monosubstituted C₁-C₈-alkyl, C₂-C₈-alkenyl, C₂-C₈-alkynyl, C₁-C₈-alkoxy, C₁-C₈-alkylamino and di(C₁-C₈-alkyl)amino, the substituents of which are selected from the group consisting of F, OH, C₁-C₆-alkoxy, phenoxy, C₁-C₆-alkylmercapto, NH₂, C₁-C₆-alkylamino and di(C₁-C₆-alkyl)amino; C₃-C₅-alkandiyl; phenyl; heteroaryl; phenyl-substituted or heteroaryl-substituted C₁-C₂-alkyl; CF₃; OH; phenoxy; benzyloxy; (C₁-C₆-alkyl)-COO; S(O)ₘ-(C₁-C₆)-alkyl which can optionally be substituted by OH or C₁-C₆-alkoxy; S(O)ₘ-phenyl; S(O)ₘ-heteroaryl; SH; phenylamino; benzylamino; (C₁-C₆-alkyl)-CONH-; (C₁-C₆-alkyl)-CON(C₁-C₄-alkyl)-; phenyl-CONH-; phenyl-CON(C₁-C₄-alkyl)-; heteroaryl-CONH-; heteroaryl-CON(C₁-C₄-alkyl)-; (C₁-C₆-alkyl)-CO-; phenyl-CO-; heteroaryl-CO-; CF₃-CO-; -OCH₂O-; -OCF₂O-; -OCH₂CH₂O-; -CH₂CH₂O-; COO(C₁-C₆-alkyl); -CONH₂; -CONH(C₁-C₆-alkyl); -CON(di(C₁-C₆-alkyl)); C(NH)-NH₂; -SO₂NH₂; -SO₂NH(C₁-C₆-alkyl); -SO₂NH(phenyl); -SO₂N(di(C₁-C₆-alkyl)); (C₁-C₆-alkyl)-SO₂NH-; (C₁-C₆-alkyl)-SO₂N(C₁-C₆-alkyl)-; phenyl-SO₂NH-; phenyl-SO₂N(C₁-C₆-alkyl)-; heteroaryl-SO₂NH-; heteroaryl-SO₂N(C₁-C₆-alkyl)-; and a residue of a saturated or at least monounsaturated aliphatic, mononuclear 5-membered to 7-membered heterocycle containing 1, 2 or 3 heteroatoms selected from the group consisting of N, O and S, which heterocycle can be substituted by one or more substituents selected from the group consisting of halogens, C₁-C₃-alkyl, C₁-C₃-alkoxy, OH, oxo and CF₃, where said heterocycle can optionally be condensed to the said group Ar; wherein all heteroaryl, phenyl, heteroaryl-containing and phenyl-containing groups, which are optionally present in the said substituents of the said group Ar, can be substituted by one or more substituents selected from the group consisting of halogens, -CN, C₁-C₃-alkyl, OH, C₁-C₃-alkoxy, and CF₃.

R⁵ is more preferably phenyl which is unsubstituted or carries one or more identical or different substituents selected from the group
consisting of: halogens; -CN; NH₂; unsubstituted and at least monosubstituted C₁-C₆-alkyl, C₂-C₆-alkenyl, C₂-C₆-alkynyl, C₁-C₃-alkoxy, C₁-C₄-alkylamino and di(C₁-C₄-alkyl)amino, the substituents of which are selected from the group consisting of F, C₁-C₃-alkoxy, C₁-C₃-alkylmercapto and NH₂; C₃-C₅-alkandiyl; phenyl; heteroaryl; phenyl-substituted or heteroaryl-substituted C₁-C₂-alkyl; CF₃; OH; (C₁-C₄-alkyl)-COO; S(O)ₘ-(C₁-C₄)-alkyl; (C₁-C₄-alkyl)-CONH-; (C₁-C₄-alkyl)-CON(C₁-C₄-alkyl)-; (C₁-C₄-alkyl)-CO-; phenyl-CO-; heteroaryl-CO-; CF₃-CO-; -OCH₂O-; -OCF₂O-; -OCH₂CH₂O-; -CH₂CH₂O-; COO(C₁-C₆-alkyl); -CONH₂; -CONH(C₁-C₄-alkyl); -CON(di(C₁-C₄-alkyl)); C(NH)NH₂; -SO₂NH₂; -SO₂NH(C₁-C₄-alkyl); -SO₂NH(phenyl); -SO₂N(di(C₁-C₄-alkyl)); (C₁-C₄-alkyl)-SO₂NH-; (C₁-C₄-alkyl)-SO₂N(C₁-C₄-alkyl)-; and a residue of a saturated or at least monounsaturated aliphatic, mononuclear 5-membered to 7-membered heterocycle containing 1, 2 or 3 heteroatoms selected from the group consisting of N, O and S, which heterocycle can be substituted by one or more substituents selected from the group consisting of halogens, C₁-C₃-alkyl, C₁-C₃-alkoxy, OH, oxo and CF₃, where said heterocycle can optionally be condensed to the said phenyl; wherein all heteroaryl, phenyl, heteroaryl-containing and phenyl-containing groups, which are optionally present in the said substituents of the said phenyl, can be substituted by one or more substituents selected from the group consisting of halogens, -CN, C₁-C₃-alkyl, OH, C₁-C₃-alkoxy, and CF₃.

R⁵ is even more preferably phenyl which is unsubstituted or carries one or more identical or different substituents selected from the group
consisting of: F; Cl; Br; C₁-C₃-alkyl; C₁-C₃-alkoxymethyl; 2-amino-3,3,3-trifluoropropyl-; CF₃; C₃-C₅-alkandiyl; phenyl; heteroaryl; benzyl; heteroaryl-methyl-; OH; C₁-C₃-alkoxy; phenoxy; trifluoromethoxy; 2,2,2-trifluoroethoxy; (C₁-C₄-alkyl)-COO; C₁-C₃-alkylmercapto; phenylmercapto; C₁-C₃-alkylsulfonyl; phenylsulfonyl; NH₂; C₁-C₄-alkylamino; di(C₁-C₄-alkyl)amino; (C₁-C₃-alkyl)-CONH-; (C₁-C₃-alkyl)-SO₂NH-; (C₁-C₃-alkyl)-CO-; phenyl-CO-; -OCH₂O-; -OCF₂O-; -CH₂CH₂O-; COO(C₁-C₄-alkyl); -CONH₂; -CONH(C₁-C₄-alkyl); -CON(di(C₁-C₄-alkyl)); -CN; -SO₂NH₂; -SO₂NH(C₁-C₄-alkyl); -SO₂N(di(C₁-C₄-alkyl)); pyrrolidinyl; piperidinyl; morpholinyl and thiomorpholinyl; wherein all heteroaryl, phenyl, heteroaryl-containing and phenyl-containing groups, which are optionally present in the said substituents of the said phenyl, can be substituted by one or more substituents selected from the group consisting of halogens, -CN, C₁-C₃-alkyl, OH, C₁-C₃-alkoxy, and CF₃.

R⁵ is most preferably selected from the group consisting of 4-fluorophenyl, 4-chlorophenyl, 4-bromophenyl, 4-(C₁-C₃-alkoxy)-phenyl, 4-trifluoromethoxyphenyl, 2-bromo-4-fluorophenyl, 2-chloro-4-fluorophenyl, 3,4-dimethylphenyl, 2,4-dimethylphenyl, 4-chloro-2-methylphenyl, 2-hydroxy-4-methylphenyl, 2-hydroxy-4-ethoxyphenyl, 2-methoxy-4-methylphenyl, 4-phenoxyphenyl, 3-fluoro-4-methylphenyl, benzo[1,3]dioxol-5-yl, 2,2-difluoro-benzo[1,3]dioxol-5-yl, 2,3-dihydrobenzofuran-5-yl, 1-methyl-3-oxo-1,2,3.4-tetrahydro-quinoxalin-6-3-dimethylamino-4-methylphenyl, 3-dimethylaminophenyl, 3-methylsulfonylamino-2-methylphenyl, 3-methylsulfonylaminophenyl, 3-(morpholin-4-yl)-phenyl, 3-(piperidin-1-yl)-phenyl, 3-(pyrrolidin-1-yl)-phenyl, 4-(2,2,2-trifluoroethoxy)phenyl, 4-chloro-3-methylsulfonylaminophenyl, 4-chloro-3-sulfamoylphenyl, 4-methyl-3-methylaminophenyl, 5-methylsulfonyl-2-methylphenyl, methylsulfanylphenyl, 4-ethylsulfanylphenyl, 3-methoxycarbonylphenyl, 4-methoxycarbonylphenyl, 3-ethoxycarbonylphenyl, 4-ethoxycarbonylphenyl, 2-bromo-4-chlorophenyl, 2,3-dichlorophenyl, bromo-2-chlorophenyl, 4-methoxyphenyl, 4-ethoxyphenyl, 3-methoxyphenyl, 3-ethoxyphenyl, trifluoromethylphenyl, 4-ethylaminophenyl, 4-methylaminophenyl, 2-aminophenyl, 4-bromo-2-fluorophenyl, 2-chlorophenyl, 3-chloro-4-methylphenyl, 4-chloro-3-methylphenyl, 2-chloro-3-methylphenyl, 2-methylphenyl, 2-acetoxy-4-methylphenyl, 2-acetoxy-4-ethoxyphenyl, 2-acetoxy-4-methoxyphenyl, 4-trifluoromethylsulfanylphenyl, naphthalen-2-yl, 1,1-dimethylindan-4-yl, 3-isobutyrylaminophenyl, 3-(2,2-dimethylpropionylamino)phenyl, 2-bromophenyl, 2-fluorophenyl.

Heteroaryl is preferably a residue of 5-membered to 10-membered, aromatic, monocyclic or bicyclic heterocycle containing 1, 2 or 3 heteroatoms selected from the group consisting of N, O and S. More preferably heteroaryl is selected from the group consisting of furyl, pyrrolyl, thienyl, thiazolyl, isothiazolyl, oxazolyl, isoxazolyl, pyrazolyl, imidazolyl, pyridazinyl, pyrazinyl, pyridinyl, pyrimidinyl, benzimidazolyl, benzothiazolyl, benzoxazolyl, quinolinyl, isoquinolinyl, quinoxalinyl, quinazolinyl, indolyl, benzofuranyl, benzodioxolyl, benzothiophenyl and indazolyl.

Aryl is preferably phenyl.

m is preferably 0 or 2.

Preferred compounds of the formula Ia are those compounds in which one or some or all of the structural moieties and groups contained therein have preferred meanings, more preferred meanings, even more preferred meanings or most preferred meanings defined above, all combinations of such preferred meanings etc. and/or of specific meanings of a group being a subject of the present invention. With respect to all preferred compounds of the formula la the present invention also includes all stereoisomeric forms and mixtures thereof in all ratios, and their pharmaceutically acceptable salts.

A compound of the formula la or a salt thereof can be prepared, for example, by a process which comprises the acylation of a heteroaromatic amine of the formula IIa with a carboxylic acid of the formula R⁵-COOH or a derivative thereof, which process also is a subject of the present invention.

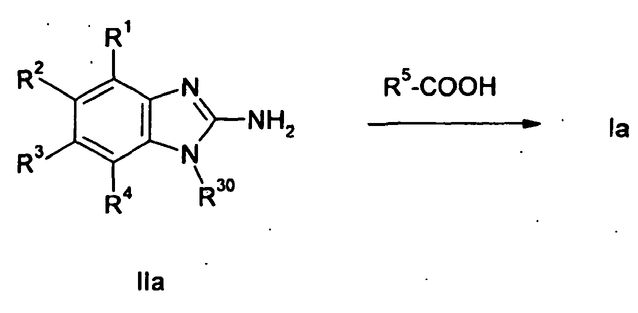

Suitable derivatives of the carboxylic acids of the formula R⁵-COOH are, for example, carboxylic acid chlorides, esters including C₁-C₄-alkyl esters, such as methyl esters or ethyl esters, optionally substituted aryl esters, such as phenyl esters or nitrophenyl esters, or activated esters, or anhydrides or mixed anhydrides. In the compounds of the formula IIa and the carboxylic acids of the formula R⁵-COOH and their derivatives the groups R¹, R², R³, R⁴, R⁵ and R³⁰ have the meanings indicated above with respect to the compounds of the formula Ia, or else functional groups can be present in protected form or in the form of a precursor. For example, when a compound of the formula la is to be prepared which contains a carboxylic acid group or an amino group, it may be appropriate that in the acylation reaction these groups are present in protected form, for example as an ester such as a tert-butyl ester or benzyl ester instead of the free carboxylic acid group, or as an acylated amino group such as a tert-butoxycarbonylamino group or benzyloxycarbonylamino group instead of the free amino group, and only subsequent to the acylation the desired final groups are liberated by deprotection. Suitable protective group strategies which may be used in the synthesis of the compounds of formula la are known to the person skilled in the art. An example of a precursor group of a functional group is the nitro group which can be converted into an amino group by reduction, for example by catalytic hydrogenation, after the acylation reaction.

The acylation reactions can be carried out under standard conditions known to the person skilled in the art. In many cases the reaction is favorably performed in an inert solvent or diluent, for example a hydrocarbon or a chlorinated hydrocarbon, such as toluene, 1,2-dichloroethane or methylene chloride, an ether, such as tetrahydrofuran, dioxane or 1,2-dimethoxyethane, an alcohol such as methanol, ethanol or isopropanol, an amide such as N,N-dimethylformamide or N-methylpyrrolidone, acetonitrile, water, or a else a mixture of two or more solvents or diluents. Depending on the individual case, it may be advantageous to perform the reaction in the presence of a base, for example an inorganic base such as sodium hydroxide, sodium carbonate or sodium hydrogencarbonate, or an organic base such as triethylamine, ethyldiisopropylamine, N-ethylmorpholine or pyridine, and/or in the presence of an acylation catalyst such as 4-dimethylaminopyridine.

If a carboxylic acid of the formula R⁵-COOH is to be used in the acylation of a compound of the formula IIa, it is often advantageous to activate the acid or a salt thereof with a condensation agent or coupling agent, for example an agent like those commonly used in peptide chemistry for the formation of amide bonds. Examples of suitable coupling agents are carbodiimides such as dicyclohexylcarbodiimide or diisopropylcarbodiimide, TOTU, i.e. O-((cyano(ethoxycarbonyl)methylene)amino)-N,N,N',N'-tetramethyluronium tetrafluoroborate, HATU, i. e. O-(7-azabenzotriazol-1-yl)-1,1,3,3-tetramethyluronium hexafluorophosphate, chloroformic acid esters like ethyl chloroformate or isobutyl chloroformate, tosyl chloride, propylphosphonic acid anhydride or carbonyldiimidazole. Depending on the individual case, the suitable reaction temperature may lie within a wide range. For example, when employing into the acylation reaction a carboxylic acid in the presence of a coupling agent or a carboxylic acid chloride, the reaction can often be carried out at room temperature.

Subsequent to the acylation reaction, besides the above-mentioned deprotection of protected groups or the conversion of a precursor group into the desired final group, optionally further functionalizations or modifications of the obtained compounds can be carried out and suitable functional groups can, for example, be esterified, amidated, transesterified, hydrolyzed, alkylated, sulfonylated, acylated, reduced, oxidized, converted into a salt, or subjected to other reactions.

The starting compounds for the preparation of the compounds of the formula la are commercially available or can be prepared according to or analogously to literature procedures. All reactions for the synthesis of the compounds of the formula la are per se well-known to the skilled person and can be carried out under standard conditions according to or analogously to procedures described in the literature, for example in Houben-Weyl, Methoden der Organischen Chemie (Methods of Organic Chemistry), Thieme-Verlag, Stuttgart, or Organic Reactions, John Wiley & Sons, New York. As mentioned above, depending on the circumstances of the individual case, in order to avoid side reactions during the synthesis of a compound of the formula la, in any reaction step it can be necessary or advantageous to temporarily block functional groups by introducing protective groups and to deprotect them in a later stage of the synthesis, or introduce functional groups in the form of precursor groups which in a later reaction step are converted into the desired functional groups. Such synthesis strategies and protective groups and precursor groups which are suitable in an individual case are known to the skilled person. If desired, the compounds of the formula la can be purified by customary purification procedures, for example by recrystallization or chromatography.

The compounds of the formula la are useful pharmaceutically active compounds which upregulate the expression of endothelial NO synthase and can be employed as medicaments for the treatment of various diseases. In the context of the present invention, treatment is understood as comprising both therapy, including alleviation and cure, of disease symptoms and prevention or prophylaxis of disease symptoms, such as, for example, the prevention of the appearance of asthmatic disease symptoms or the prevention of myocardial infarction or of myocardial reinfarction in relevant patients. The diseases or disease symptoms can be acute or chronic. Diseases which can be treated with the compounds of the formula Ia include, for example, cardiovascular diseases like stable and unstable angina pectoris, coronary heart disease, Prinzmetal angina (spasm), acute coronary syndrome, heart failure, myocardial infarction, stroke, thrombosis, peripheral artery occlusive disease (PAOD), endothelial dysfunction, atherosclerosis, restenosis, endothel damage after PTCA, hypertension including essential hypertension, pulmonary hypertension and secondary hypertension (renovascular hypertension, chronic glomerulonephritis), erectile dysfunction and ventricular arrhythmia. Further, the compounds of the formula la lower the cardiovascular risk of postmenopausal women and of women taking contraceptives. Compounds of the formula la can additionally be used in the treatment, i. e. the therapy and prevention, of diabetes and diabetes complications (nephropathy, retinopathy), angiogenesis, asthma bronchiale, chronic renal failure, cirrhosis of the liver, osteoporosis, restricted memory performance or a restricted ability to learn. Preferred indications are stable angina pectoris, coronary heart disease, hypertension, endothelial dysfunction, atherosclerosis and diabetes complications.

The compounds of the formula Ia can be used in combination with other pharmaceutically active compounds, preferably with compounds which are able to enhance the effect of the compounds of the formula Ia. Examples of such other compounds include statins; ACE inhibitors; AT1 antagonists; argininase inhibitors; PDE V inhibitors; calcium antagonists; alpha blockers; beta blockers; thiamazole (methimazole) and analogous compounds; arginine; tetrahydrobiopterin; vitamins, in particular vitamin C and vitamin B6; niacine.

The compounds of the formula la and their pharmaceutically acceptable salts, optionally in combination with other pharmaceutically active compounds, can be administered to animals, preferably to mammals, and in particular to humans, as pharmaceuticals by themselves, in mixtures with one another or in the form of pharmaceutical preparations. Further subjects of the present invention therefore also are the compounds of the formula la and their pharmaceutically acceptable salts for use as pharmaceuticals, their use as transcription stimulating agents or upregulating agents of endothelial NO synthase in conditions in which an increased expression of said enzyme or an increased NO level or the normalization of a decreased NO level in a patient is desired, and in particular their use in the treatment, i. e. the therapy and prevention, of the above-mentioned syndromes, as well as their use for preparing medicaments for these purposes. Furthermore, a subject of the present invention are pharmaceutical preparations (or pharmaceutical compositions) which comprise an effective dose of at least one compound of the formula la and/or a pharmaceutically acceptable salt thereof and a pharmaceutically acceptable carrier, i.e. one or more pharmaceutically acceptable carrier substances or vehicles and/or additives or excipients.

Thus, for example, a subject of the present invention are acylamino-substituted heteroaromatic compounds of the formula la, which are defined as above, in any of their stereoisomeric forms and mixtures thereof in any ratio, and the pharmaceutically acceptable salts thereof, for use in the treatment of cardiovascular diseases, stable or unstable angina pectoris, coronary heart disease, Prinzmetal angina, acute coronary syndrome, heart failure, myocardial infarction, stroke, thrombosis, peripheral artery occlusive disease, endothelial dysfunction, atherosclerosis, restenosis, endothel damage after PTCA, hypertension, essential hypertension, pulmonary hypertension, secondary hypertension, renovascular hypertension, chronic glomerulonephritis, erectile dysfunction, ventricular arrhythmia, diabetes, diabetes complications, nephropathy, retinopathy, angiogenesis, asthma bronchiale, chronic renal failure, cirrhosis of the liver, osteoporosis, restricted memory performance or a restricted ability to learn, or for the lowering of cardiovascular risk of postmenopausal women or of women taking contraceptives, and their use as pharmaceutical as well as pharmaceutical preparations which comprise an effective dose of at least one compound of the formula la and/or a pharmaceutically acceptable salt thereof and a pharmaceutically acceptable carrier. With respect to compounds of the formula la for use as pharmaceutical or for use in the treatment of the beforementioned diseases all explanations given above with respect to the compounds of the formula la per se likewise apply. Thus, a further subject of the invention also are compounds of the formula la for use as pharmaceutical or for use in the treatment of the beforementioned diseases, in which one or more, including all, of the groups and numbers in the definition of the compounds have preferred meanings, more preferred meanings, even more preferred meanings or most preferred meanings or any specific meaning.

The pharmaceuticals according to the invention can be administered orally, for example in the form of pills, tablets, lacquered tablets, sugar-coated tablets, granules, hard and soft gelatin capsules, aqueous, alcoholic or oily solutions, syrups, emulsions or suspensions, or rectally, for example in the form of suppositories. Administration can also be carried out parenterally, for example subcutaneously, intramuscularly or intravenously, in the form of solutions for injection or infusion. Other suitable administration forms are, for example, percutaneous or topical administration, for example in the form of ointments, tinctures, sprays or transdermal therapeutic systems, or the inhalative administration in the form of nasal sprays or aerosol mixtures, or, for example, microcapsules, implants or rods. The preferred administration form depends, among others, on the disease to be treated and on its severity.

The amount of a compound of the formula la and/or its pharmaceutically acceptable salts in the pharmaceutical preparations normally ranges from about 0.2 to about 800 mg, preferably from about 0.5 to about 500 mg, in particular from about 1 to about 200 mg, per dose, but depending on the type of the pharmaceutical preparation it may also be higher. The pharmaceutical preparations usually comprise from about 0.5 to about 90 % by weight of the compounds of the formula la and/or their pharmaceutically acceptable salts. The production of the pharmaceutical preparations can be carried out in a manner known per se. To this end, one or more compounds of the formula la and/or their pharmaceutically acceptable salts, together with one or more solid or liquid pharmaceutical carrier substances and/or additives (or auxiliary substances) and, if desired, in combination with other pharmaceutically active compounds having therapeutic or prophylactic action, are brought into a suitable administration form or dosage form which can then be used as a pharmaceutical in human or veterinary medicine.

For the production of pills, tablets, sugar-coated tablets and hard gelatin capsules it is possible to use, for example, lactose, starch, for example maize starch, or starch derivatives, talc, stearic acid or its salts, etc. Soft gelatin capsules and suppositories can comprise, for example, fats, waxes, semisolid and liquid polyols, natural or hardened oils, etc. Suitable carrier substances for the preparation of solutions, for example of solutions for injection, or of emulsions or syrups are, for example, water, physiologically sodium chloride solution, alcohols such as ethanol, glycerol, polyols, sucrose, invert sugar, glucose, mannitol, vegetable oils, etc. It is also possible to lyophilize the compounds of the formula la and their pharmaceutically acceptable salts and to use the resulting lyophilisates, for example, for preparing preparations for injection or infusion. Suitable carriers for microcapsules, implants or rods are, for example, copolymers of glycolic acid and lactic acid.

Besides the compound or compounds of the invention and carrier substances, the pharmaceutical preparations can also contain additives such as, for example, fillers, disintegrants, binders, lubricants, wetting agents, stabilizers, emulsifiers, dispersants, preservatives, sweeteners, colorants, flavorings, aromatizers, thickeners, diluents, buffer substances, solvents, solubilizers, agents for achieving a depot effect, salts for altering the osmotic pressure, coating agents or antioxidants.

The dosage of the compound of the formula la to be administered and/or of a pharmaceutically acceptable salt thereof depends on the individual case and is, as is customary, to be adapted to the individual circumstances to achieve an optimum effect. Thus, it depends on the nature and the severity of the disorder to be treated, and also on the sex, age, weight and individual responsiveness of the human or animal to be treated, on the efficacy and duration of action of the compounds used, on whether the use is for the therapy of a acute or chronic disease or prophylactic, or on whether other active compounds are administered in addition to compounds of the formula Ia. In general, a daily dose of from about 0.01 to about 100 mg/kg, preferably from about 0.1 to about 10 mg/kg, in particular from about 0.3 to about 5 mg/kg (in each case mg per kg of bodyweight) is appropriate for administration to an adult weighing approximately 75 kg in order to obtain the desired results. The daily dose can be administered in a single dose or, in particular when larger amounts are administered, be divided into several, for example two, three or four individual doses. In some cases, depending on the individual response, it may be necessary to deviate upwards or downwards from the given daily dose.

The compounds of the formula Ia can also be used for other purposes than those indicated in the foregoing. Non-limiting examples include diagnostic purposes, such as the use in the examination of cell or tissue samples, the use as biochemical tools and the use as intermediates for the preparation of further compounds, e.g. pharmaceutically active compounds.

### Examples

### General procedure for acylations

66 mg (0.543 mmol) of 4-dimethylaminopyridine, 84 mg (0.543 mmol) of N-(3-dimethylaminopropyl)-N'-ethyl-carbodiimide and 73 mg (0.543 mmol) of 1-hydroxybenzotriazole were added at 0°C to 0.543 mmol of the respective carboxylic acid dissolved in 2 ml of dry dimethylformamide, and the mixture was stirred for 20 min. 0.543 mmol of the respective heteroaromatic amino compound were then added and the mixture was stirred for 12 h at room temperature. The product isolated by aqueous work-up was purified by preparative HPLC (RP-18; acetonitrile/water + 0.1 % trifluoroacetic acid or formic acid).

In the following listings of example compounds, besides the mass number of the (M+H)⁺ peak in the mass spectra (MS) obtained from the prepared compound, chromatographic parameters of the prepared compound are given, namely Rf values in the case of characterization by thin layer chromatography (TLC) and retention times (RT; in minutes) in the case of characterization by HPLC. The following chromatographic methods were applied.

### Method A (HPLC)

Column: Merck Lichrocart 55-2, Purospher Star, RP 18 e; temperature: 40 °C; flow: 0.750 ml/min; solvent A: acetonitrile/water 90/10 + 0.5 % formic acid; solvent B: acetonitrile/water 10/90 + 0.5 % formic acid; gradient: time 0.00 min: 5 % solvent A + 95 % solvent B, time 0.50 min: 5 % solvent A + 95 % solvent B, time 1.75 min: 95 % solvent A + 5 % solvent B, time 4.25 min: 95 % solvent A + 5 % solvent B, time 4.50 min: 5 % solvent A + 95 % solvent B, time 5.00 min: 5 % solvent A + 95 % solvent B.

### Method B (HPLC)

Column: YMC J'Sphere ODS H80, 33 x 2 mm, 4 µ; temperature: 30 °C; flow: 1.000 ml/min; solvent A: acetonitrile + 0.05 % formic acid; solvent B: water + 0.05 % formic acid; gradient: time 0.00 min: 10 % solvent A + 90 % solvent B, time 2.50 min: 95 % solvent A + 5 % solvent B, time 3.30 min: 95 % solvent A + 5 % solvent B, time 3.35 min: 10 % solvent A + 90 % solvent B.

### Method C (HPLC)

Column: Merck Purospher Star, 55 x 2 mm, 3 µ; temperature: room temperature; flow: 0.45 ml/min; solvent A: acetonitrile + 0.1 % formic acid; solvent B: water + 0.1 % formic acid; gradient: time 0.00 min: 5 % solvent A + 95 % solvent B, time 5.00 min: 95 % solvent A + 5 % solvent B, time 7.00 min: 95 % solvent A + 5 % solvent B, time 8.00 min: 5 % solvent A + 95 % solvent B.

### Method D (HPLC)

Column: Merck Lichrocart 55-2, Purospher Star, RP 18 e; temperature: 40°C; flow: 1.000 ml/min; solvent A: acetonitrile/water 90/10+ 0.5 % formic acid; solvent B: acetonitrile/water 10/90 + 0.5 % formic acid; gradient: time 0.00 min: 5 % solvent A + 95 % solvent B, time 0.75 min: 95 % solvent A + 5 % solvent B, time 3.00 min: 95 % solvent A + 5 % solvent B, time 3.20 min: 5 % solvent A + 95 % solvent B, time 4.00 min: 5 % solvent A + 95 % solvent B.

### Method E (TLC)

Silica gel; dichloromethane/methanol 98/2.

### Method F (TLC)

Silica gel; heptane/ethyl acetate 3/1.

The compounds of the formula Ig listed in Table 1 are reference examples.

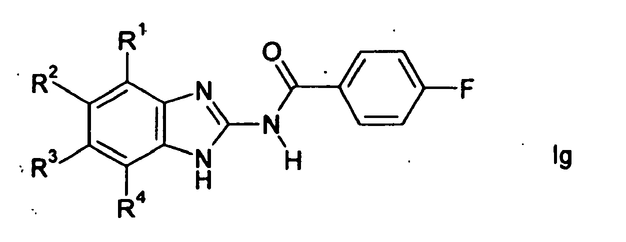

**Table 1. Reference examples of formula lg**

| Ex. no. | R¹ | R² | R³ | R⁴ | MS (M+H)⁺ | HPLC/ TLC | Method |
|---|---|---|---|---|---|---|---|
| 1 | H | H | H | H | 256 | 0.16 | E |
| 4 | H | H | Cl | H | 290 | 0.17 | E |
| 5 | CH₃ | H | H | H | 270 | 0.33 | F |
| 6 | H | CH₃ | CH₃ | H | 284 | | |
| 9 | O₂N | H | H | H | 301 | 3.041 | A |
| 13 | H | CH₃ | H | H | 270 | 2.749 | A |
| 17 | H | F | H | H | 274 | 2.885 | A |
| 20 | H | F | Cl | H | 308 | 3.057 | A |
| 21 | H | CH₃O | H | H | 286 | 2,692 | A |
| 22 | CH₃ | CH₃ | H | H | 284 | 2.931 | A |
| 23 | H | (CH₃)₃C | H | H | 312 | 3.035 | A |
| 25 | H | F | F | H | 292 | 2.957 | A |
| 29 | H | CH₃ | Cl | H | 304 | 3.030 | A |
| 33 | F | H | F | H | 292 | 3.009 | A |
| 34 | O₂N | CH₃ | H | H | 315 | 3.106 | A |

### Example 47

### 4-Fluoro-N-(1-methyl-5-trifluoromethyl-1H-benzimidazol-2-yl)-benzamide

MS: m/e = 338 (M+H)⁺. HPLC: RT = 3.111 min (method A).

Example compounds of the formula Ih are listed in Table 2.

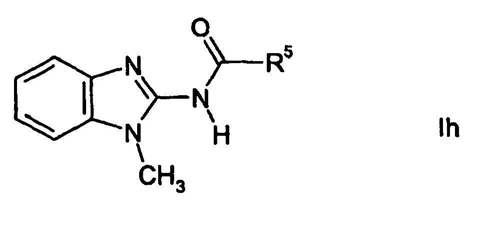

**Table 2. Example compounds of formula Ih**

| Ex. no. | R⁵ | MS (M+H)⁺ | HPLC | Method |
|---|---|---|---|---|
| 48 | 4-fluorophenyl | 270 | 4.16 | C |
| 56 | 3-methylsulfonylamino-4-methylphenyl | 359 | 1.94 | B |
| 57 | 2,4-dimethylphenyl | 280 | 3.79 | C |
| 58 | 2,4-difluorophenyl | 288 | 4.37 | C |
| 60 | 4-methylsulfanylphenyl | 298 | 4.68 | C |
| 63 | 3-methylsulfonylaminophenyl | 345 | 3.91 | C |
| 68 | 3-(piperidin-1-yl)phenyl (a) | 335 | 3.21 | C |
| 69 | 3-(4-methylpiperazin-1-yl)phenyl (a) | 350 | 2.83 | C |
| 70 | 3-(morpholin-4-yl)phenyl (a) | 337 | 3.96 | C |
| 71 | 3-(pyridin-4-ylamino)phenyl (a) | 344 | 2.97 | C |
| 85 | 3-dimethylaminophenyl (a) | 295 | 3.37 | C |
| 86 | 2,3-dichlorophenyl | 320 | 4.68 | C |

| | | | | |
|---|---|---|---|---|
| (a) The compound was obtained as salt with trifluoroacetic acid. | | | | |

The compounds of the formula Ik listed in Table 3 are reference examples.

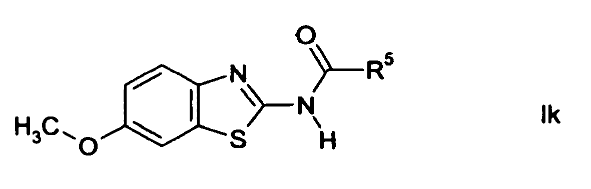

**Table 3. Reference examples of formula Ik**

| Ex. no. | R⁵ | MS (M+H)⁺ | HPLC | Method |
|---|---|---|---|---|
| 90 | 3-dimethylaminophenyl | 328 | 1.89 | D |
| 91 | 3,4-dimethylphenyl | 313 | 3.21 | A |
| 99 | 2,3-difluorophenyl | 321 | 1.95 | D |
| 103 | 3-acetylaminophenyl | 342 | 1.82 | D |
| 107 | 4-isopropoxyphenyl | 342 | 2.10 | D |

### Determination of activation of eNOS transcription

Activation of eNOS transcription was determined as described in detail in Li et al., "Activation of protein kinase C alpha and/or epsilon enhances transcription of the human endothelial nitric oxide synthase gene", Mol. Pharmacol. 53 (1998) 630.

Briefly, a 3.5kB long fragment 5' of the starting codon of the eNOS gene was cloned, sequenced and cloned in firefly luciferase expression plasmids to monitor activation of the eNOS promoter by reporter gene activity. A human endothelial cell line stable transfected and expressing this promoter-reporter construct was used for compound testing. Cells were incubated for 18 h with compounds.

All compounds were dissolved in sterile dimethyl sulfoxide (DMSO). A final concentration of 0.5% DMSO in complete medium was allowed. Induction of reporter gene expression in these cells was measured using a standard luciferase assay system (Promega, Cat. No E150) according to the manufacturer's instructions. Luciferase induction in cells incubated with compounds were compared to those incubated with solvent alone. The ratio of both activities (transcription induction ratio, TIR) was plotted as a function of compound concentration. Typically, TIR values started at low concentrations at a ratio of 1, indicating no compound effect, and extended up to a maximum TIR value TIR(max) which indicates the increase of the eNOS transcription. EC₅₀ values of transcription induction ratios as a function of compound concentration were determined graphically.

The effect of compounds on eNOS-transcription was confirmed in a second assay based on eNOS protein detection. Primary human umbilical vein cord endothelial cells (HUVEC) were isolated and cultivated according to standard procedures. Confluent cells were incubated with compounds for 18 h and the effect on eNOS protein expression determined by a quantitative Western blotting procedure. After compounds incubation, HUVEC were lysed in ice-cold lysis buffer containing 10 mM Tris-HCl, pH 8.0, 1 % SDS and protease inhibitors. The lysate was subjected to a standard denaturating polyacrylamide gel electropheresis and blotted to nitrocellulose membranes. Using a specific primary monoclonal antibody (Transduction Laboratories, UK) and alkaline phosphatase labelled secondary antibody (Jackson Labs), a specific eNOS protein band was visualized and quantified based on a chemifluorescence detection method.

The results obtained with example compounds of the invention and reference examples are listed in Table 4.

**Table 4. EC₅₀ values of transcription induction ratio**

| Compound of Example No. | EC₅₀ [µM] |
|---|---|
| 1 (reference example) | 0.028 |
| 4 (reference example) | <0.1 |
| 5 (reference example) | 0.35 |
| 6 (reference example) | 0.31 |
| 9 (reference example) | 0.050 |
| 13 (reference example) | 0.065 |
| 17 (reference example) | <0.1 |
| 20 (reference example) | <0.1 |
| 21 (reference example) | 0.29 |
| 22 (reference example) | 0.086 |
| 23 (reference example) | 0.048 |
| 25 (reference example) | <0.1 |
| 29 (reference example) | 0.084 |
| 33 (reference example) | 0.079 |
| 34 (reference example) | 0.18 |
| 48 | 0.21 |
| 90 (reference example) | 12 |
| 91 (reference example) | 0.34 |
| 99 (reference example) | 0.020 |
| 103 (reference example) | 0.016 |
| 107 (reference example) | 0.84 |

The effect of the compounds of the invention can also be investigated in the following animal models (animal experiments are performed in accordance to the German animal protection law and to the guidelines for the use of experimental animals as given by the Guide for the Care and Use of Laboratory Animals of the US National Institutes of Health).

### Animals and Treatment (Experiments A - C)

ApoE and eNOS deficient mice (C57BL/6J background, Jackson Laboratory, Bar Harbor, Me) are used. All animals are 10 to 12 weeks of age and weigh 22 to 28 g. Three days before surgery mice are divided into 4 groups (apoE control, n = 10 to 12; apoE with test compounds, n = 10 to 12; eNOS control, n = 10 to 12; eNOS with test compounds, n = 10 to 12) and receive either a standard rodent chow (containing 4 % fat and 0.001 % cholesterol; in the following designated as placebo group) or a standard rodent chow + test compound (10 or 30 mg/kg/d p.o.).

### A. Anti-hypertensive effect in ApoE knockout mice

Blood-pressure is determined in conscious mice using a computerized tail-cuff system (Visitech Systems, Apex, Nc). After treatment of ApoE deficient mice and eNOS deficient mice with the test compounds the blood pressure is compared to the results obtained with a placebo treatment.

### B. Inhibition of neointima formation and atherogenesis (femoral artery cuff)

After 3 day treatment of ApoE deficient mice with the respective compound, (10 mg/kg/d pressed in chow), animals are anesthetized with an intraperitoneal injection of pentobarbital (60 mg/kg) followed by an intramuscular injection of xylazin (2 mg/kg) and a cuff is placed around the femoral artery as described in Moroi et al.(J. Clin. Invest. 101 (1998) 1225). Briefly, the left femoral artery is dissected. A non-occlusive 2.0 mm polyethylene cuff made of PE-50 tubing (inner diameter 0.56 mm, outer diameter 0.965 mm, Becton Dickinson, Mountain View, Ca) is placed around the artery and tied in place with two 7-0 sutures. The right femoral artery is isolated from the surrounding tissues but a cuff is not placed. Treatment with the respective compound is continued for 14 days after surgery. Then the animals are sacrificed. The aorta are taken for determination of vascular eNOS expressions by quantitative western blotting. Both femoral arteries are harvested, fixed in formalin and embedded in paraffin. 20 cross sections (10 µm) are cut from the cuffed portion of the left femoral artery and from the corresponding segment of the right artery. Sections are subjected to standard hematoxylin and eosin staining. Morphometric analyses are performed using an image analysis computer program (LeicaQWin, Leica Imaging Systems, Cambridge, GB). For each cross section the area of the lumen, the neointima and the media are determined. To this end, the neointima is defined as the area between the lumen and the internal elastic lamina and the media is defined as the area between the internal and the external elastic lamina. The ratio between the area of the neointima and the area of the media is expressed as the neointima/media ratio. The results obtained in the compound group are compared to those obtained in the placebo group.

### C. Prevention of atherosclerotic plaque formation in chronic treatment

ApoE deficient mice are treated for 16 weeks with the respective compound pressed in chow and finally sacrificed. Aortas are removed from each mouse, fixed in formalin and embedded in paraffin. Plaque formation is measured via lipid lesions formation in the aortas (from aortic arch to diaphragm) and is analyzed by oil red O staining. For quantifying the effect of the respective compound on vascular eNOS expression the femoral arteries are used in this experiment. The results obtained in the compound group are compared to those obtained in the placebo group.

### D. Improvement of coronary function in diseased ApoE deficient mice

Old Male wild-type C57BL/6J mice (Charles River Wiga GmbH, Sulzfeld), and apoE deficient mice (C57BL/6J background, Jackson Laboratory, Bar Harbor, Me) 6 month of age and weighing 28 to 36 g are used in the experiments. Mice are divided into 3 groups (C57BU6, n = 8; apoE control, n = 8; apoE with respective compound, n = 8) and receive for 8 weeks either a standard rodent chow (containing 4 % fat and 0.001 % cholesterol) or a standard rodent chow + respective compound (30 mg/kg/d p.o.). Mice are anesthetized with sodium pentobarbitone (100 mg/kg i.p.), and the hearts are rapidly excised and placed into ice-cold perfusion buffer. The aorta is cannulated and connected to a perfusion apparatus (Hugo Sachs Electronics, Freiburg, Germany) which is started immediately at a constant perfusion pressure of 60 mm Hg. Hearts are perfused in a retrograde fashion with modified Krebs bicarbonate buffer, equilibrated with 95 % O₂ and 5 % CO₂ and maintained at 37.5 °C.
A beveled small tube (PE 50) is passed through a pulmonary vein into the left ventricle and pulled through the ventricular wall, anchored in the apex by a fluted end, and connected to a tip-micromanometer (Millar 1.4 French). The left atrium is cannulated through the same pulmonary vein and the heart switched to the working mode with a constant preload pressure of 10 mm Hg and an afterload pressure of 60 mm Hg. Aortic outflow and atrial inflow are continuously measured using ultrasonic flow probes (HSE/Transonic Systems Inc.). Coronary flow is calculated as the difference between atrial flow and aortic flow. All hemodynamic data are digitized at a sampling rate of 1000 Hz and recorded with a PC using spezialized software (HEM, Notocord).

Hearts are allowed to stabilize for 30 min. All functional hemodynamic data are measured during steady state, and during volume- and pressure loading.
Left ventricular function curves are constructed by varying pre-load pressure. For acquisition of preload curves, afterload is set at 60 mm Hg and preload is adjusted in 5 mm Hg steps over a range of 5 to 25 mm Hg. Hearts are allowed to stabilize at baseline conditions between pressure- and volume-loading.

## Claims

1. A compound of the formula la, 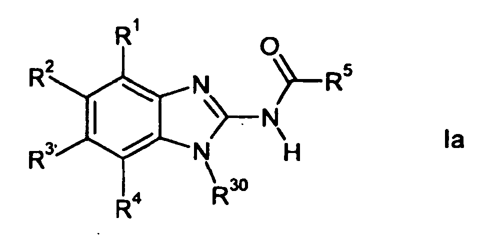 in any of its stereoisomeric forms or a mixture thereof in any ratio, or a pharmaceutically acceptable salt thereof, wherein in the formula la:
R¹ and R⁴ are independently from each other selected from the group consisting of: H; unsubstituted and at least monosubstituted C₁-C₁₀-alkyl, C₂-C₁₀-alkenyl and C₂-C₁₀-alkynyl, the substituents of which are selected from the group consisting of F, OH, C₁-C₈-alkoxy, C₁-C₈-alkylmercapto, -CN, COOR⁶, CONR⁷R⁸, and unsubstituted and at least monosubstituted phenyl and heteroaryl where the substituents of the phenyl and heteroaryl groups are selected from the group consisting of halogens, -CN, C₁-C₃-alkyl, C₁-C₃-alkoxy and CF₃; unsubstituted and at least monosubstituted phenyl and heteroaryl the substituents of which are selected from the group consisting of halogens, -CN, C₁-C₃-alkyl, C₁-C₃-alkoxy and CF₃; COR⁹; CONR¹⁰R¹¹; COOR¹²; CF₃; halogens; -CN; NR¹³R¹⁴; OR¹⁵; S(O)ₘR¹⁶; SO₂NR¹⁷R¹⁸; and NO₂;
R² and R³ are independently from each other selected from the group consisting of: H; halogens; -CN; unsubstituted and at least monosubstituted C₁-C₁₀-alkyl the substituents of which are selected from the group consisting of OH, phenyl, and heteroaryl; OH; C₁-C₁₀-alkoxy; phenoxy; S(O)ₘR¹⁹; CF_{3;} -CN; NO₂; C₁-C₁₀-alkylamino; di(C₁-C₁₀-alkyl)amino; (C₁-C₆-alkyl)-CONH-; unsubstituted and at least monosubstituted phenyl-CONH- and phenyl-SO₂-O- the substituents of which are selected from the group consisting of halogens, -CN, methyl and methoxy; C₁-C₆-alkyl-SO₂-O-; unsubstituted and at least monosubstituted (C₁-C₆-alkyl)-CO- the substituents of which are selected from the group consisting of F, di(C₁-C₃-alkyl)amino, pyrrolidinyl and piperidinyl; and phenyl-CO- the phenyl part of which is unsubstituted or at least monosubstituted by substituents selected from the group consisting of C₁-C₃-alkyl, halogens and methoxy;
R⁵ is a group Ar which is unsubstituted or carries one or more identical or different substituents selected from the group consisting of:
halogens; -CN; NH₂; unsubstituted and at least monosubstituted C₁-C₁₀-alkyl, C₂-C₁₀-alkenyl, C₂-C₁₀-alkynyl, C₁-C₁₀-alkoxy, C₁-C₁₀-alkylamino and di(C₁-C₁₀-alkyl)amino, the substituents of which are selected from the group consisting of F, OH, C₁-C₈-alkoxy, aryloxy, C₁-C₈-alkylmercapto, NH₂, C₁-C₈-alkylamino and di(C₁-C₈-alkyl)amino; C₃-C₅-alkandiyl; phenyl; heteroaryl; aryl-substituted or heteroaryl-substituted C₁-C₄-alkyl; CF₃; OH; phenoxy; benzyloxy; (C₁-C₁₀-alkyl)-COO-; S(O)ₘR²⁰; SH; phenylamino; benzylamino; (C₁-C₁₀-alkyl)-CONH-; (C₁-C₁₀₋alkyl)-CO-N(C₁-C₄-alkyl)-; phenyl-CONH-; phenyl-CO-N(C₁-C₄-alkyl)-; heteroaryl-CONH-; heteroaryl-CO-N(C₁-C₄-alkyl)-; (C₁-C₁₀-alkyl)-CO-; phenyl-CO-; heteroaryl-CO-; CF₃-CO-; -OCH₂O-; -OCF₂O-; -OCH₂CH₂O-; -CH₂CH₂O-; COOR²¹; CONR²²R²³; C(NH)-NH₂; SO₂NR²⁴R²⁵ ; R²⁶SO₂NH-; R²⁷SO²N(C₁-C₆-alkyl)-; and a residue of a saturated or at least monounsaturated aliphatic, monocyclic 5-membered to 7-membered heterocycle containing 1, 2 or 3 heteroatoms selected from the group consisting of N, O and S, which heterocycle can be substituted by one or more substituents selected from the group consisting of halogens, C₁-C₃-alkyl, C₁-C₃-alkoxy, OH, oxo and CF₃, where said heterocycle can optionally be condensed to the said group Ar; wherein all aryl, heteroaryl, phenyl, aryl-containing, heteroaryl-containing and phenyl-containing groups, which are optionally present in the said substituents of the said group Ar, can be substituted by one or more substituents selected from the group consisting of halogens, -CN, C₁-C₃-alkyl, OH, C₁-C₃-alkoxy, and CF₃;
R⁶ is selected from the group consisting of:
H; C₁-C₁₀-alkyl which can be substituted by one or more substituents selected from the group consisting of F, C₁-C₈-alkoxy and di(C₁-C₈-alkyl)amino; aryl-(C₁-C₄-alkyl)-and heteroaryl-(C₁-C₄-alkyl)- both of which can be substituted by one or more substituents selected from the group consisting of halogens, C₁-C₄-alkyl, C₁-C₄-alkoxy and di(C₁-C₆-alkyl)amino;
R⁷ is selected from the group consisting of:
H; C₁-C₁₀-alkyl which can be substituted by one or more substituents selected from the group consisting of F, C₁-C₈-alkoxy, di(C₁-C₈-alkyl)amino and phenyl; phenyl; indanyl; and heteroaryl; wherein each of the aromatic groups can be unsubstituted or carry one or more substituents selected from the group consisting of halogens, -CN, C₁-C₃-alkyl, C₁-C₃-alkoxy and CF₃;
R⁸ is H or C₁-C₁₀-alkyl;
R⁹ is selected from the group consisting of:
C₁-C₁₀-alkyl which can be substituted by one or more substituents from the group consisting of F, C₁-C₄-alkoxy and di(C₁-C₃-alkyl)amino; and unsubstituted and at least monosubstituted phenyl and heteroaryl the substituents of which are selected from the group consisting of C₁-C₃-alkyl, C₁-C₃-alkoxy, halogens; -CN and CF₃;
R¹⁰, independently from R⁷, is defined as R⁷;
R¹¹, independently from R⁸, is defined as R⁸;
R¹², independently from R⁶, is defined as R⁶;
R¹³ is selected from the group consisting of:
H; C₁-C₆-alkyl; unsubstituted and substituted phenyl, benzyl, heteroaryl, (C₁-C₆-alkyl)-CO-, phenyl-CO-, and heteroaryl-CO-, the substituents of which are selected from the group consisting of halogens, -CN, C₁-C₃-alkyl, C₁-C₃-alkoxy and CF₃, wherein one or more of these substituents can be present;
R¹⁴, independently from R¹³, is defined as R¹³;
R¹⁵ is selected from the group consisting of:
H; C₁-C₁₀-alkyl; (C₁-C₃-alkoxy)-C₁-C₃-alkyl- and substituted and unsubstituted benzyl, phenyl and heteroaryl, the substituents of which are selected from the group consisting of halogens, -CN, C₁-C₃-alkyl, C₁-C₃-alkoxy and CF₃, wherein one or more of these substituents can be present;
R¹⁶ is selected from the group consisting of:
C₁-C₁₀-alkyl which can be substituted by one or more substituents selected from the group consisting of F, OH, C₁-C₈-alkoxy, aryloxy, C₁-C₈-alkylmercapto, C₁-C₈-alkylamino and di(C₁-C₈-alkyl)amino; CF₃; and substituted and unsubstituted phenyl and heteroaryl, the substituents of which are selected from the group consisting of halogens, -CN, C₁-C₃-alkyl, C₁-C₃-alkoxy and CF₃, wherein one or more of these substitutents can be present;
R¹⁷, independently from R⁷, is defined as R⁷;
R¹⁸, independently from R⁸, is defined as R⁸;
R¹⁹, independently from R¹⁶, is defined as R¹⁶;
R²⁰, independently from R¹⁶, is defined as R¹⁶;
R²¹, independently from R⁶, is defined as R⁶;
R²², independently from R⁷, is defined as R⁷;
R²³, independently from R⁸, is defined as R⁸;
R²⁴, independently from R⁷, is defined as R⁷;
R²⁵, independently from R⁸, is defined as R⁸;
R²⁶, independently from R¹⁶, is defined as R¹⁶;
R²⁷, independently from R¹⁶, is defined as R¹⁶;
R³⁰ is methyl;
heteroaryl is a residue of a 5-membered to 10-membered, aromatic, monocyclic or bicyclic heterocycle containing one or more heteroatoms selected from the group consisting of N, O and S;
aryl is phenyl, naphth-1-yl or naphth-2-yl;
the group Ar is phenyl, naphth-1-yl or naphth-2-yl;
m is 0, 1 or 2;
provided that R⁵ cannot be phenyl, 2-chlorophenyl, 4-bromophenyl, 4-methoxyphenyl or 3,4-methylenedioxyphenyl if simultaneously R¹, R², R³ and R⁴ are all hydrogen; and that R⁵ cannot be phenyl, 4-methylphenyl or 4-methoxyphenyl if simultaneously R¹, R³ and R⁴ are all hydrogen and R² is chloro.

2. A compound of the formula la as defined in claim 1, in any of its stereoisomeric forms or a mixture thereof in any ratio, or a pharmaceutically acceptable salt thereof, wherein R¹ and R⁴ are independently from one another selected from the group consisting of H, halogens and C₁-C₄-alkyl, and R² and R³ are independently from one another selected from the group consisting of H, halogens and C₁-C₄-alkyl.

3. A compound of the formula la as defined in claim 1 and/or 2, in any of its stereoisomeric forms or a mixture thereof in any ratio, or a pharmaceutically acceptable salt thereof, wherein one or two of the groups R¹, R², R³ and R⁴ are different from hydrogen.

4. A compound of the formula la as defined in in claim 1 and/or 2, in any of its stereoisomeric forms or a mixture thereof in any ratio, or a pharmaceutically acceptable salt thereof, wherein R¹, R², R³ and R⁴ are all H.

5. A compound of the formula Ia as defined in one or more of claims 1 to 4, in any of its stereoisomeric forms or a mixture thereof in any ratio, or a pharmaceutically acceptable salt thereof, wherein R⁵ is phenyl which is unsubstituted or carries one or more identical or different substituents selected from the group consisting of:
halogens; -CN; NH₂; unsubstituted and at least monosubstituted C₁-C₆-alkyl, C₂-C₆-alkenyl, C₂-C₆-alkynyl, C₁-C₃-alkoxy, C₁-C₄-alkylamino and di(C₁-C₄-alkyl)amino, the substituents of which are selected from the group consisting of F, C₁-C₃-alkoxy, C₁-C₃-alkylmercapto and NH₂; C₃-C₅-alkandiyl; phenyl; heteroaryl; phenyl-substituted or heteroaryl-substituted C₁-C₂-alkyl; CF₃; OH; (C₁-C₄-alkyl)-COO; S(O)ₘ-(C₁-C₄)-alkyl; (C₁-C₄-alkyl)-CONH-; (C₁-C₄-alkyl)-CON(C₁-C₄-alkyl)-; (C₁-C₄-alkyl)-CO-; phenyl-CO-; heteroaryl-CO-; CF₃-CO-; -OCH₂O-; -OCF₂O-; -OCH₂CH₂O-; -CH₂CH₂O-; COO(C₁-C₆-alkyl); -CONH₂; -CONH(C₁-C₄-alkyl); -CON(di(C₁-C₄-alkyl)); C(NH)NH₂; -SO₂NH₂; -SO₂NH(C₁-C₄-alkyl); -SO₂NH(phenyl); -SO₂N(di(C₁-C₄-alkyl)); (C₁-C₄-alkyl)-SO₂NH-; (C₁-C₄-alkyl)-SO₂N(C₁-C₄-alkyl)-; and a residue of a saturated or at least monounsaturated aliphatic, mononuclear 5-membered to 7-membered heterocycle containing 1, 2 or 3 heteroatoms selected from the group consisting of N, O and S, which heterocycle can be substituted by one or more substituents selected from the group consisting of halogens, C₁-C₃-alkyl, C₁-C₃-alkoxy, OH, oxo and CF₃, where said heterocycle can optionally be condensed to the said phenyl, wherein all heteroaryl, phenyl, heteroaryl-containing and phenyl-containing groups, which are optionally present in the said substituents of the said phenyl, can be substituted by one or more substituents selected from the group consisting of halogens, -CN, C₁-C₃-alkyl, OH, C₁-C₃-alkoxy, and CF₃.

6. A compound of the formula la as defined in one or more of claims 1 to 5, in any of its stereoisomeric forms or a mixture thereof in any ratio, or a pharmaceutically acceptable salt thereof, wherein R⁵ is phenyl which is unsubstituted or carries one or more identical or different substituents selected from the group consisting of: F; Cl; Br; C₁-C₃-alkyl; C₁-C₃-alkoxymethyl; 2-amino-3,3,3-trifluoropropyl-; CF₃; C₃-C₅-alkandiyl; phenyl; heteroaryl; benzyl; heteroaryl-methyl-; OH; C₁-C₃-alkoxy; phenoxy; trifluoromethoxy; 2,2,2-trifluoroethoxy; (C₁-C₄-alkyl)-COO; C₁-C₃-alkylmercapto; phenylmercapto; C₁-C₃-alkylsulfonyl; phenylsulfonyl; NH₂; C₁-C₄-alkylamino; di(C₁-C₄-alkyl)amino; (C₁-C₃-alkyl)-CONH-; (C₁-C₃-alkyl)-SO₂NH-; (C₁-C₃-alkyl)-CO-; phenyl-CO-; -OCH₂O-; -OCF₂O-; -CH₂CH₂O-; COO(C₁-C₄-alkyl); -CONH₂; -CONH(C₁-C₄-alkyl); -CON(di(C₁-C₄-alkyl)); -CN; -SO₂NH₂; -SO₂NH(C₁-C₄-alkyl); -SO₂N(di(C₁-C₄-alkyl)); pyrrolidinyl; piperidinyl; morpholinyl and thiomorpholinyl; wherein all heteroaryl, phenyl, heteroaryl-containing and phenyl-containing groups, which are optionally present in the said substituents of the said phenyl, can be substituted by one or more substituents selected from the group consisting of halogens, -CN, C₁-C₃-alkyl, OH, C₁-C₃-alkoxy, and CF₃.

7. A compound of the formula Ia as defined in one or more of claims 1 to 6, in any of its stereoisomeric forms or a mixture thereof in any ratio, or a pharmaceutically acceptable salt thereof, for use as pharmaceutical.

8. A pharmaceutical preparation, comprising an effective dose of at least one compound of the formula la as defined in one or more of claims 1 to 6, in any of its stereoisomeric forms or a mixture thereof in any ratio, and/or a pharmaceutically acceptable salt thereof and a pharmaceutically acceptable carrier.

9. The use of a compound of the formula la as defined in one or more of claims 1 to 6, in any of its stereoisomeric forms or a mixture thereof in any ratio, or a pharmaceutically acceptable salt thereof, for the manufacture of a medicament for the treatment of cardiovascular diseases, stable or unstable angina pectoris, coronary heart disease, Prinzmetal angina, acute coronary syndrome, heart failure, myocardial infarction, stroke, thrombosis, peripheral artery occlusive disease, endothelial dysfunction, atherosclerosis, restenosis, endothel damage after PTCA, hypertension, essential hypertension, pulmonary hypertension, secondary hypertension, renovascular hypertension, chronic glomerulonephritis, erectile dysfunction, ventricular arrhythmia, diabetes, diabetes complications, nephropathy, retinopathy, angiogenesis, asthma bronchiale, chronic renal failure, cirrhosis of the liver, osteoporosis, restricted memory performance or a restricted ability to learn, or for the lowering of cardiovascular risk of postmenopausal women or of women taking contraceptives.

## Patentansprüche

1. Verbindung der Formel Ia, 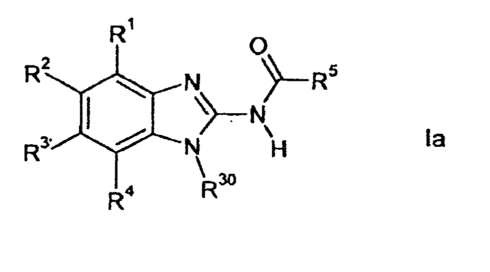 in einer beliebigen stereoisomeren Form oder eine Mischung davon in einem beliebigen Verhältnis, oder ein pharmazeutisch akzeptables Salz davon, wobei in der Formel Ia:
R¹ und R⁴ unabhängig voneinander ausgewählt sind aus der Gruppe bestehend aus:
H; unsubstituiertem und wenigstens einfach substituiertem C₁-C₁₀-Alkyl, C₂-C₁₀-Alkenyl und C₂-C₁₀-Alkinyl, wobei die Substituenten ausgewählt sind aus der Gruppe bestehend aus F, OH, C₁-C₈-Alkoxy, C₁-C₈-Alkylmercapto, -CN, COOR⁶, CONR⁷R⁸, und unsubstituiertem und wenigstens einfach substituiertem Phenyl und Heteroaryl, wobei die Substituenten der Phenyl- und Heteroarylgruppen ausgewählt sind aus der Gruppe bestehend aus Halogenen, -CN, C₁-C₃-Alkyl, C₁-C₃-Alkoxy und CF₃; unsubstituiertem und wenigstens einfach substituiertem Phenyl und Heteroaryl, wobei die Substituenten ausgewählt sind aus der Gruppe bestehend aus Halogenen, -CN, C₁-C₃-Alkyl, C₁-C₃-Alkoxy und CF₃; COR⁹; CONR¹⁰R¹¹; COOR¹²; CF₃; Halogenen; -CN; NR¹³R¹⁴ ; OR¹⁵; S(O)ₘR¹⁶; SO₂NR¹⁷R¹⁸; und NO₂;
R² und R³ unabhängig voneinander ausgewählt sind aus der Gruppe bestehend aus:
H; Halogenen; -CN; unsubstituiertem und wenigstens einfach substituiertem C₁-C₁₀-Alkyl, wobei die Substituenten ausgewählt sind aus der Gruppe bestehend aus OH, Phenyl und Heteroaryl; OH; C₁-C₁₀-Alkoxy; Phenoxy; S(O)ₘR¹⁹; CF₃; -CN; NO₂; C₁-C₁₀-Alkylamino; Di(C₁-C₁₀-alkyl)amino; (C₁-C₆-Alkyl) -CONH-; unsubstituiertem und wenigstens einfach substituiertem Phenyl-CONH- und Phenyl-SO₂-O-, wobei die Substituenten ausgewählt sind aus der Gruppe bestehend aus Halogenen, -CN, Methyl und Methoxy; C₁-C₆-Alkyl-SO₂-O-; unsubstituiertem und wenigstens einfach substituiertem (C₁-C₆-Alkyl)-CO-, wobei die Substituenten ausgewählt sind aus der Gruppe bestehend aus F, Di(C₁-C₃-alkyl)amino, Pyrrolidinyl und Piperidinyl; und Phenyl-CO-, wobei der Phenylteil unsubstituiert oder wenigstens einfach substituiert durch Substituenten ausgewählt aus der Gruppe bestehend aus C₁-C₃-Alkyl, Halogenen und Methoxy ist;
R⁵ für eine Gruppe Ar steht, die unsubstituiert ist oder einen oder mehrere gleiche oder verschiedene Substituenten trägt, ausgewählt aus der Gruppe bestehend aus:
Halogenen; -CN; NH₂; unsubstituiertem und wenigstens einfach substituiertem C₁-C₁₀-Alkyl, C₂-C₁₀-Alkenyl, C₂-C₁₀-Alkinyl, C₁-C₁₀-Alkoxy, C₁-C₁₀-Alkylamino und Di(C₁-C₁₀-alkyl)amino, wobei die Substituenten ausgewählt sind aus der Gruppe bestehend aus F, OH, C₁-C₈-Alkoxy, Aryloxy, C₁-C₈-Alkylmercapto, NH₂, C₁-C₈-Alkylamino und Di(C₁-C₈-alkyl)amino; C₃-C₅-Alkandiyl; Phenyl; Heteroaryl; arylsubstituiertem oder heteroarylsubstituiertem C₁-C₄-Alkyl; CF₃; OH; Phenoxy; Benzyloxy; (C₁-C₁₀-Alkyl)-COO-; S(O)ₘR²⁰; SH; Phenylamino; Benzylamino; (C₁-C₁₀-Alkyl)-CONH-; (C₁-C₁₀-Alkyl)-CO-N (C₁-C₉-alkyl)-; Phenyl-CONH-; Phenyl-CO-N(C₁-C₄-alkyl)-; Heteroaryl-CONH-; Heteroaryl-CO-N(C₁-C₄-alkyl)-; (C₁-C₁₀-Alkyl)-CO-; Phenyl-CO-; Heteroaryl-CO-; CF₃-CO-; -OCH₂O-; -OCF₂O-; -OCH₂CH₂O-; -CH₂CH₂O-; COOR²¹; CONR²²R²³; C(NH)-NH₂; SO₂NR²⁴R²⁵; R²⁶SO₂NH-; R²⁷SO₂N(C₁-C₆-Alkyl)-; und einem Rest eines gesättigten oder wenigstens einfach ungesättigten aliphatischen, monocyclischen fünfgliedrigen bis siebengliedrigen Heterocyclus mit 1, 2 oder 3 Heteroatomen ausgewählt aus der Gruppe bestehend aus N, O und S, wobei der Heterocyclus substituiert sein kann durch einen oder mehrere Substituenten ausgewählt aus der Gruppe bestehend aus Halogenen, C₁-C₃-Alkyl, C₁-C₃-Alkoxy, OH, Oxo und CF₃, wobei der Heterocyclus gegebenenfalls an die Gruppe Ar kondensiert sein kann; wobei alle Arylgruppen, Heteroarylgruppen, Phenylgruppen, arylhaltigen Gruppen, heteroarylhaltigen Gruppen und phenylhaltigen Gruppen, die gegebenenfalls in den Substituenten der Gruppe Ar vorhanden sind, substituiert sein können durch einen oder mehrere Substituenten ausgewählt aus der Gruppe bestehend aus Halogenen, -CN, C₁-C₃-Alkyl, OH, C₁-C₃-Alkoxy, und CF₃;
R⁶ ausgewählt ist aus der Gruppe bestehend aus:
H; C₁-C₁₀-Alkyl, das substituiert sein kann durch einen oder mehrere Substituenten ausgewählt aus der Gruppe bestehend aus F, C₁-C₈-Alkoxy und Di(C₁-C₈-alkyl)amino; Aryl-(C₁-C₄-alkyl)-und Heteroaryl-(C₁-C₄-alkyl)-, die beide substituiert sein können durch einen oder mehrere Substituenten ausgewählt aus der Gruppe bestehend aus Halogenen, C₁-C₄-Alkyl, C₁-C₄-Alkoxy und Di(C₁-C₆-alkyl)amino;
R⁷ ausgewählt ist aus der Gruppe bestehend aus:
H; C₁-C₁₀-Alkyl, das substituiert sein kann durch einen oder mehrere Substituenten ausgewählt aus der Gruppe bestehend aus F, C₁-C₈-Alkoxy, Di(C₁-C₈-alkyl)amino und Phenyl; Phenyl; Indanyl; und Heteroaryl; wobei die aromatischen Gruppen jeweils unsubstituiert sein können oder einen oder mehrere Substituenten tragen, ausgewählt aus der Gruppe bestehend aus Halogenen, -CN, C₁-C₃-Alkyl, C₁-C₃-Alkoxy und CF₃;
R⁸ für H oder C₁-C₁₀-Alkyl steht;
R⁹ ausgewählt ist aus der Gruppe bestehend aus:
C₁-C₁₀-Alkyl, das substituiert sein kann durch einen oder mehrere Substituenten ausgewählt aus der Gruppe bestehend aus F, C₁-C₄-Alkoxy und Di(C₁-C₃-alkyl)amino; und unsubstituiertem und wenigstens einfach substituiertem Phenyl und Heteroaryl, wobei die Substituenten ausgewählt sind aus der Gruppe bestehend aus C₁-C₃-Alkyl, C₁-C₃-Alkoxy, Halogenen, -CN und CF₃;
R¹⁰ unabhängig von R⁷ wie R⁷ definiert ist;
R¹¹ unabhängig von R⁸ wie R⁸ definiert ist;
R¹² unabhängig von R⁶ wie R⁶ definiert ist;
R¹³ ausgewählt ist aus der Gruppe bestehend aus:
H; C₁-C₆-Alkyl; unsubstituiertem und substituiertem Phenyl, Benzyl, Heteroaryl, (C₁-C₆-Alkyl)-CO-, Phenyl-CO- und Heteroaryl-CO-, wobei die Substituenten ausgewählt sind aus der Gruppe bestehend aus Halogenen, -CN, C₁-C₃-Alkyl, C₁-C₃-Alkoxy und CF₃, wobei einer oder mehrere dieser Substituenten vorhanden sein können;
R¹⁴ unabhängig von R¹³ wie R¹³ definiert ist;
R¹⁵ ausgewählt ist aus der Gruppe bestehend aus:
H; C₁-C₁₀-Alkyl; (C₁-C₃-Alkoxy)-C₁-C₃-alkyl- und substituiertem und unsubstituiertem Benzyl, Phenyl und Heteroaryl, wobei die Substituenten ausgewählt sind aus der Gruppe bestehend aus Halogenen, -CN, C₁-C₃-Alkyl, C₁-C₃-Alkoxy und CF₃, wobei einer oder mehrere dieser Substituenten vorhanden sein können;
R¹⁶ ausgewählt ist aus der Gruppe bestehend aus:
C₁-C₁₀-Alkyl, das substituiert sein kann durch einen oder mehrere Substituenten ausgewählt aus der Gruppe bestehend aus F, OH, C₁-C₈-Alkoxy, Aryloxy, C₁-C₈-Alkylmercapto, C₁-C₈-Alkylamino und Di(C₁-C₈-alkyl)amino; CF₃; und substituiertem und unsubstituiertem Phenyl und Heteroaryl, wobei die Substituenten ausgewählt sind aus der Gruppe bestehend aus Halogenen, -CH, C₁-C₃-Alkyl, C₁-C₃-Alkoxy und CF₃, wobei einer oder mehrere dieser Substituenten vorhanden sein können;
R¹⁷ unabhängig von R⁷ wie R⁷ definiert ist;
R¹⁸ unabhängig von R⁸ wie R⁸ definiert ist;
R¹⁹ unabhängig von R¹⁶ wie R¹⁶ definiert ist;
R²⁰ unabhängig von R¹⁶ wie R¹⁶ definiert ist;
R²¹ unabhängig von R⁶ wie R⁶ definiert ist;
R²² unabhängig von R⁷ wie R⁷ definiert ist;
R²³ unabhängig von R⁸ wie R⁸ definiert ist;
R²⁴ unabhängig von R⁷ wie R⁷ definiert ist;
R²⁵ unabhängig von R⁸ wie R⁸ definiert ist;
R²⁶ unabhängig von R¹⁶ wie R¹⁶ definiert ist;
R²⁷ unabhängig von R¹⁶ wie R¹⁶ definiert ist;
R³⁰ für Methyl steht;
Heteroaryl für einen Rest eines fünfgliedrigen bis zehngliedrigen aromatischen monocyclischen oder bicyclischen Heterocyclus mit einem oder mehreren Heteroatomen, ausgewählt aus der Gruppe bestehend aus N, 0 und S, steht;
Aryl für Phenyl, Naphth-1-yl oder Naphth-2-yl steht;
die Gruppe Ar für Phenyl, Naphth-1-yl oder Naphth-2-yl steht;
m für 0, 1 oder 2 steht;
mit der Maßgabe, daß R⁵ nicht für Phenyl, 2-Chlorphenyl, 4-Bromphenyl, 4-Methoxyphenyl oder 3,4-Methylendioxyphenyl stehen kann, wenn gleichzeitig R¹, R², R³ und R⁴ alle für Wasserstoff stehen; und daß R⁵ nicht für Phenyl, 4-Methylphenyl oder 4-Methoxyphenyl stehen kann, wenn gleichzeitig R¹, R³ und R⁹ alle für Wasserstoff stehen und R² für Chlor steht.

2. Verbindung der Formel Ia nach Anspruch 1, in einer beliebigen stereoisomeren Form oder eine Mischung davon in einem beliebigen Verhältnis, oder ein pharmazeutisch akzeptables Salz davon, wobei R¹ und R⁴ unabhängig voneinander ausgewählt sind aus der Gruppe bestehend aus H, Halogenen und C₁-C₄-Alkyl und R² und R³ unabhängig voneinander ausgewählt sind aus der Gruppe bestehend aus H, Halogenen und C₁-C₄-Alkyl.

3. Verbindung der Formel Ia nach Anspruch 1 und/oder 2, in einer beliebigen stereoisomeren Form oder eine Mischung davon in einem beliebigen Verhältnis, oder ein pharmazeutisch akzeptables Salz davon, wobei eine oder zwei der Gruppen R¹, R², R³ und R⁴ nicht für Wasserstoff stehen.

4. Verbindung der Formel Ia nach Anspruch 1 und/oder 2, in einer beliebigen stereoisomeren Form oder eine Mischung davon in einem beliebigen Verhältnis, oder ein pharmazeutisch akzeptables Salz davon, wobei R¹, R², R³ und R⁴ alle für H stehen.

5. Verbindung der Formel Ia nach einem oder mehreren der Ansprüche 1 bis 4, in einer beliebigen stereoisomeren Form oder eine Mischung davon in einem beliebigen Verhältnis, oder ein pharmazeutisch akzeptables Salz davon, wobei R⁵ für Phenyl steht, das unsubstituiert ist oder einen oder mehrere gleiche oder verschiedene Substituenten trägt, ausgewählt aus der Gruppe bestehend aus:
Halogenen; -CN; NH₂; unsubstituiertem und wenigstens einfach substituiertem C₁-C₆-Alkyl, C₂-C₆-Alkenyl, C₂-C₆-Alkinyl, C₁-C₃-Alkoxy, C₁-C₄-Alkylamino und Di(C₁-C₄-alkyl)amino, wobei die Substituenten ausgewählt sind aus der Gruppe bestehend aus F, C₁-C₃-Alkoxy, C₁-C₃-Alkylmercapto und NH₂; C₃-C₅-Alkandiyl; Phenyl; Heteroaryl; phenylsubstituiertem oder heteroarylsubstituiertem C₁-C₂-Alkyl; CF₃; OH; (C₁-C₄-Alkyl)-COO; S(O)ₘ-(C₁-C₄)-Alkyl; (C₁-C₄)-Alkyl-CONH-; (C₁-C₄-Alkyl)-CON (C₁-C₄-alkyl) -; (C₁-C₄-Alkyl) -CO-; Phenyl-CO-; Heteroaryl-CO-; CF₃-CO-; -OCH₂O-; -OCF₂O-; -OCH₂CH₂O-; -CH₂CH₂O-; COO(C₁-C₆-Alkyl); -CONH₂; -CONH(C₁-C₉-Alkyl); -CON(Di(C₁-C₄-alkyl)); C(NH)NH₂; -SO₂NH₂; -SO₂NH(C₁-C₄-Alkyl); -SO₂NH(Phenyl); -SO₂N(Di(C₁-C₄-alkyl)) ; (C₁-C₄-Alkyl)-SO₂NH-; (C₁-C₄-Alkyl)-SO₂N(C₁-C₄-alkyl)-; und einem Rest eines gesättigten oder wenigstens einfach ungesättigten aliphatischen, einkernigen fünfgliedrigen bis siebengliedrigen Heterocyclus mit 1, 2 oder 3 Heteroatomen ausgewählt aus der Gruppe bestehend aus N, O und S, wobei der Heterocyclus substituiert sein kann durch einen oder mehrere Substituenten ausgewählt aus der Gruppe bestehend aus Halogenen, C₁-C₃-Alkyl, C₁-C₃-Alkoxy, OH, Oxo und CF₃, wobei der Heterocyclus gegebenenfalls an das Phenyl kondensiert sein kann, wobei alle Heteroarylgruppen, Phenylgruppen, heteroarylhaltigen Gruppen und phenylhaltigen Gruppen, die gegebenenfalls in den Substituenten des Phenyl vorhanden sind, substituiert sein können durch einen oder mehrere Substituenten ausgewählt aus der Gruppe bestehend aus Halogenen, -CN, C₁-C₃-Alkyl, OH, C₁-C₃-Alkoxy und CF₃.

6. Verbindung der Formel Ia nach einem oder mehreren der Ansprüche 1 bis 5, in einer beliebigen stereoisomeren Form oder eine Mischung davon in einem beliebigen Verhältnis, oder ein pharmazeutisch akzeptables Salz davon, wobei R⁵ für Phenyl steht, das unsubstituiert ist oder einen oder mehrere gleiche oder verschiedene Substituenten trägt, ausgewählt aus der Gruppe bestehend aus:
F; Cl; Br; C₁-C₃-Alkyl; C₁-C₃-Alkoxymethyl; 2-Amino-3,3,3-trifluorpropyl-; CF₃; C₃-C₅-Alkandiyl; Phenyl; Heteroaryl; Benzyl; Heteroarylmethyl-; OH; C₁-C₃-Alkoxy; Phenoxy; Trifluormethoxy; 2,2,2-Trifluorethoxy; (C₁-C₄-Alkyl)-COO; C₁-C₃-Alkylmercapto; Phenylmercapto; C₁-C₃-Alkylsulfonyl; Phenylsulfonyl; NH₂; C₁-C₄-Alkylamino; Di(C₁-C₄-alkyl)amino, (C₁-C₃-Alkyl)-CONH-; (C₁-C₃-Alkyl)-SO₂NH-; (C₁-C₃-Alkyl)-CO-; Phenyl-CO-; -OCH₂O-; -OCF₂O-; -CH₂CH₂O-; COO(C₁-C₄-Alkyl); -CONH₂; -CONH(C₁-C₄-Alkyl); -CON(Di(C₁-C₄-alkyl)); -CN; -SO₂NH₂; -SO₂NH(C₁-C₄-Alkyl); -SO₂N(Di(C₁-C₄-alkyl)); Pyrrolidinyl; Piperidinyl; Morpholinyl und Thiomorpholinyl; wobei alle Heteroarylgruppen, Phenylgruppen, heteroarylhaltigen Gruppen und phenylhaltigen Gruppen, die gegebenenfalls in den Substituenten des Phenyl vorhanden sind, substituiert sein können durch einen oder mehrere Substituenten ausgewählt aus der Gruppe bestehend aus Halogenen, -CN, C₁-C₃-Alkyl, OH, C₁-C₃-Alkoxy und CF₃.

7. Verbindung der Formel Ia nach einem oder mehreren der Ansprüche 1 bis 6, in einer beliebigen stereoisomeren Form oder eine Mischung davon in einem beliebigen Verhältnis, oder ein pharmazeutisch akzeptables Salz davon, zur Verwendung als Arzneimittel.

8. Pharmazeutische Zubereitung, enthaltend eine wirksame Dosis wnigstens einer wie in einem oder mehreren der Ansprüche 1 bis 6 definierten Verbindung der Formel Ia, in einer beliebigen stereoisomeren Form oder eine Mischung davon in einem beliebigen Verhältnis, und/oder ein pharmazeutisch akzeptables Salz davon und einen pharmazeutisch akzeptablen Träger.

9. Verwendung einer wie in einem oder mehreren der Ansprüche 1 bis 6 definierten Verbindung der Formel Ia, in einer beliebigen stereoisomeren Form oder eine Mischung davon in einem beliebigen Verhältnis, oder eines pharmazeutisch akzeptablen Salzes davon, zur Herstellung eines Medikaments zur Behandlung von Herz-Kreislauf-Erkrankungen, stabiler oder instabiler Angina pectoris, koronarer Herzkrankheit, Prinzmetal-Angina, akutem Koronarsyndrom, Herzinsuffizienz, Myokardinfarkt, Schlaganfall, Thrombose, peripherer arterieller Verschlußkrankheit, Endotheldysfunktion, Atherosklerose, Restenose, Endothelschaden nach PTCA, Hypertonie, essentieller Hypertonie, pulmonaler Hypertonie, sekundärer Hypertonie, renovaskulärer Hypertonie, chronischer Glomerulonephritis, erektiler Dysfunktion, ventrikulärer Arrhythmie, Diabetes, diabetischen Komplikationen, Nephropathie, Retinopathie, Angiogenese, Bronchialasthma, chronischem Nierenversagen, Leberzirrhose, Osteoporose, eingeschränkter Gedächtnisleistung oder einer eingeschränkten Lernfähigkeit, oder zur Senkung des Herz-Kreislauf-Risikos von postmenopausalen Frauen oder Frauen, die empfängnisverhütende Mittel einnehmen.

## Revendications

1. Composé de la formule Ia, 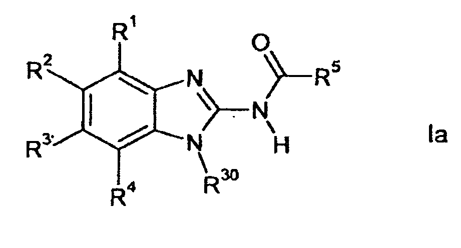 selon l'une ou l'autre de ses formes stéréoisomères ou dans un mélange de celles-ci selon n'importe quelle proportion, ou un sel pharmaceutiquement acceptable de celui-ci, dans laquelle formule Ia :
R¹ et R⁴ sont chacun indépendamment l'un de l'autre choisis parmi le groupe constitué de :
un atome H ; les groupes alkyle en C₁ à C₁₀, alcényle en C₂ à C₁₀ et alcynyle en C₂ à C₁₀ non substitués et au moins monosubstitués, dont les substituants sont choisis parmi le groupe constitué de l'atome F, des groupes OH, alcoxy en C₁ à C₈, alkyle en C₁ à C₈-mercapto, -CN, COOR⁶, CONR⁷R⁸, et des groupes phényle et hétéroaryle non substitués et au moins monosubstitués dans lesquels les substituants des groupes phényle et hétéroaryle sont choisis parmi le groupe constitué d'halogènes, des groupes -CN, alkyle en C₁ à C₃, alcoxy en C₁ à C₃ et CF₃; des groupes phényle et hétéroaryle non substitués et au moins monosubstitués, dont les substituants sont choisis parmi le groupe constitué des halogènes, des groupes -CN, alkyle en C₁ à C₃, alcoxy en C₁ à C₃ et CF₃ ; un groupe COR⁹ ; un groupe CONR¹⁰R¹¹ ; un groupe COOR¹² ; un groupe CF₃ ; des halogènes ; un groupe -CN ; un groupe NR¹³R¹⁴ ; un groupe OR¹⁵ ; un groupe S(O)ₘR¹⁶ ; un groupe SO₂NR¹⁷R¹⁸; et un groupe NO₂;
R² et R³ sont chacun indépendamment l'un de l'autre choisis parmi le groupe constitué de :
un atome H ; des halogènes ; un groupe -CN ; un groupe alkyle en C₁ à C₁₀ non substitué et au moins monosubstitué, dont les substituants sont choisis parmi le groupe constitué des groupes OH, phényle et hétéroaryle ; un groupe OH ; un groupe alcoxy en C₁ à C₁₀ ; un groupe phénoxy ; un groupe S(O)ₘR¹⁹; un groupe CF₃ ; un groupe -CN ; un groupe NO₂; un groupe alkyle en C₁ à C₁₀-amino ; un groupe di (alkyle en C₁ à C₁₀)amino ; un groupe (alkyle en C₁ à C₆)-CONH- ; des groupes phényl-CONH- et phényl-SO₂-O- non substitués et au moins monosubstitués, dont les substituants sont choisis parmi le groupe constitué d'halogènes, des groupes -CN, méthyle et méthoxy ; un groupe alkyle en C₁ à C₆-SO₂-O- ; un groupe (alkyle en C₁ à C₆)-CO- non substitué et au moins monosubstitué dont les substituants sont choisis parmi le groupe constitué de l'atome F, des groupes di(alkyle en C₁ à C₃)amino, pyrrolidinyle et pipéridinyle ; et un groupe phényl-CO-, dont la partie phényle est non substituée ou au moins monosubstituée par des substituants choisis parmi le groupe constitué d'un groupe alkyle en C₁ à C₃, des halogènes et un groupe méthoxy ;
R⁵ est un groupe Ar qui est non substitué ou qui porte un ou plusieurs substituants identiques ou différents choisis parmi le groupe constitué de :
des halogènes ; un groupe -CN ; un groupe NH₂ ; des groupes alkyle en C₁ à C₁₀, alcényle en C₂ à C₁₀, alcynyle en C₂ à C₁₀, alcoxy en C₁ à C₁₀, alkyle en C₁ à C₁₀-amino et di (alkyle en C₁ à C₁₀)amino non substitués et au moins monosubstitués, dont les substituants sont choisis parmi le groupe constitué de l'atome F, des groupes OH, alcoxy en C₁ à C₈, aryloxy, alkyle en C₁ à C₈-mercapto, NH₂, alkyle en C₁ à C₈-amino et di(alkyle en C₁ à C₈)amino ; un groupe alcanediyle en C₃ à C₅ ; un groupe phényle ; un groupe hétéroaryle ; un groupe alkyle en C₁ à C₄ à substitution aryle ou à substitution hétéroaryle ; un groupe CF₃ ; un groupe OH ; un groupe phénoxy ; un groupe benzyloxy ; un groupe (alkyle en C₁ à C₁₀)-COO- ; un groupe S(O)ₘR²⁰; un groupe SH ; un groupe phénylamino ; un groupe benzylamino ; un groupe (alkyle en C₁ à C₁₀)-CONH-; un groupe (alkyle en C₁ à C₁₀)-CO-N(alkyle en C₁ à C₄) - ; un groupe phényl-CONH- ; un groupe phényl-CO-N(alkyle en C₁ à C₄) - ; un groupe hétéroaryl-CONH- ; un groupe hétéroaryl-CO-N(alkyle en C₁ à C₄)- ; un groupe (alkyle en C₁ à C₁₀)-CO- ; un groupe phényl-CO- ; un groupe hétéroaryl-CO- ; un groupe CF₃-CO- ; un groupe -OCH₂O- ; un groupe -OCF₂O- ; un groupe -OCH₂CH₂O- ; un groupe -CH₂CH₂O- ; un groupe COOR²¹ ; un groupe CONR²²R²³ ; un groupe C(NH)-NH₂ ; un groupe SO₂NR²⁹R²⁵ ; un groupe R²⁶SO₂NH- ; un groupe R²⁷SO₂N (alkyle en C₁ à C₆)- ; et un résidu d'un hétérocycle aliphatique monocyclique de cinq chaînons à sept chaînons, saturé ou au moins monoinsaturé, comprenant 1, 2 ou 3 hétéroatomes choisis parmi le groupe constitué des atomes N, O et S, lequel hétérocycle peut être substitué par un ou plusieurs substituants choisis parmi le groupe constitué d'halogènes, des groupes alkyle en C₁ à C₃, alcoxy en C₁ à C₃, OH, oxo et CF₃, où ledit hétérocycle peut éventuellement être condensé en ledit groupe Ar ; dans lequel tous les groupes aryle, hétéroaryle, phényle, contenant un groupe aryle, contenant un groupe hétéroaryle et contenant un groupe phényle, lesquels sont éventuellement présents dans lesdits substituants dudit groupe Ar, peuvent être substitués par un ou plusieurs substituants choisis parmi le groupe constitué d'halogènes, des groupes -CN, alkyle en C₁ à C₃, OH, alcoxy en C₁ à C₃ et CF₃;
R⁶ est choisi parmi le groupe constitué de:
un atome H ; un groupe alkyle en C₁ à C₁₀ qui peut être substitué par un ou plusieurs substituants choisis parmi le groupe constitué de l'atome F, des groupes alcoxy en C₁ à C₈ et di(alkyle en C₁ à C₈)amino ; des groupes aryl-(alkyle en C₁ à C₄)- et hétéroaryl-(alkyle en C₁ à C₄) -, les deux pouvant être substitués par un ou plusieurs substituants choisis parmi le groupe constitué d'halogènes, des groupes alkyle en C₁ à C₄, alcoxy en C₁ à C₄ et di(alkyle en C₁ à C₆)amino ;
R⁷ est choisi parmi le groupe constitué de :
un atome H ; un groupe alkyle en C₁ à C₁₀ qui peut être substitué par un ou plusieurs substituants choisis parmi le groupe constitué de l'atome F, des groupes alcoxy en C₁ à C₈, di (alkyle en C₁ à C₈)amino et phényle ; un groupe phényle ; un groupe indanyle ; et un groupe hétéroaryle ; dans lequel chacun des groupes aromatiques peut être non substitué ou porter un ou plusieurs substituants choisis parmi le groupe constitué d'halogènes, des groupes -CN, alkyle en C₁ à C₃, alcoxy en C₁ à C₃ et CF₃;
R⁸ est un atome d'hydrogène ou un groupe alkyle en C₁ à C₁₀;
R⁹ est choisi parmi le groupe constitué de :
un groupe alkyle en C₁ à C₁₀ qui peut être substitué par un ou plusieurs substituants choisis parmi le groupe constitué de l'atome F, des groupes alcoxy en C₁ à C₄ et di(alkyle en C₁ à C₃)amino ; et des groupes phényle et hétéroaryle non substitués et au moins monosubstitués, dont les substituants sont choisis parmi le groupe constitué des groupes alkyle en C₁ à C₃, alcoxy en C₁ à C₃, -CN, CF₃ et d'halogènes ;
R¹⁰, indépendamment de R⁷, est défini comme R⁷ ;
R¹¹, indépendamment de R⁸, est défini comme R⁸ ;
R¹², indépendamment de R⁶, est défini comme R⁶ ;
R¹³ est choisi parmi le groupe constitué de :
un atome H ; un groupe alkyle en C₁ à C₆ ; des groupes phényle, benzyle, hétéroaryle, (alkyle en C₁ à C₆)-CO-, phényl-CO- et hétéroaryl-CO- non substitués et substitués, dont les substituants sont choisis parmi le groupe constitué d'halogènes, des groupes -CN, alkyle en C₁ à C₃, alcoxy en C₁ à C₃ et CF₃, dans lequel un ou plusieurs de ces substituants peuvent être présents ;
R¹⁴, indépendamment de R¹³, est défini comme R¹³ ;
R¹⁵ est choisi parmi le groupe constitué de :
un atome H ; un groupe alkyle en C₁ à C₁₀ ; un groupe (alcoxy en C₁ à C₃)-C₁-C₃-alkyle et des groupes benzyle, phényle et hétéroaryle, dont les substituants sont choisis parmi le groupe constitué d'halogènes, des groupes -CN, alkyle en C₁ à C₃, alcoxy en C₁ à C₃ et CF₃, dans lequel un ou plusieurs de ces substituants peuvent être présents ;
R¹⁶ est choisi parmi le groupe constitué de :
un groupe alkyle en C₁ à C₁₀ qui peut être substitué par un ou plusieurs substituants choisis parmi le groupe constitué de l'atome F, des groupes OH, alcoxy en C₁ à C₈, aryloxy, alkyle en C₁ à C₈-mercapto, alkyle en C₁ à C₈-amino et di(alkyle en C₁ à C₈)amino ; un groupe CF₃ ; et des groupes phényle et hétéroaryle substitués et non substitués, dont les substituants sont choisis parmi le groupe constitué d'halogènes, des groupes -CN, alkyle en C₁ à C₃, alcoxy en C₁ à C₃ et CF₃, dans lequel un ou plusieurs de ces substituants peuvent être présents ;
R¹⁷, indépendamment de R⁷, est défini comme R⁷ ;
R¹⁸, indépendamment de R⁸, est défini comme R⁸ ;
R¹⁹, indépendamment de R¹⁶, est défini comme R¹⁶ ;
R²⁰, indépendamment de R¹⁶, est défini comme R¹⁶ ;
R²¹, indépendamment de R⁶, est défini comme R⁶ ;
R²², indépendamment de R⁷, est défini comme R⁷;
R²³, indépendamment de R⁸, est défini comme R⁸ ;
R²⁴, indépendamment de R⁷, est défini comme R⁷ ;
R²⁵, indépendamment de R⁸, est défini comme R⁸ ;
R²⁶, indépendamment de R¹⁶, est défini comme R¹⁶ ;
R²⁷, indépendamment de R¹⁶, est défini comme R¹⁶ ;
R³⁰ représente un groupe méthyle ;
le groupe hétéroaryle est un résidu d'un hétérocycle aromatique, monocyclique ou bicyclique avec de cinq chaînons à dix chaînons, comprenant d'un ou plusieurs hétéroatomes choisis parmi le groupe constitué des atomes N, O et S ;
le groupe aryle est un groupe phényle, un groupe naptht-1-yl ou un groupe naptht-2-yl ;
le groupe Ar est un groupe phényle, un groupe naptht-1-yl ou un groupe naptht-2-yl ;
m vaut 0, 1 ou 2 ;
à condition que R⁵ ne soit pas un groupe phényle, un groupe 2-chlorophényle, un groupe 4-bromophényle, un groupe 4-méthoxyphényle ou un groupe 3,4-méthylènedioxyphényle, si R¹, R², R³ et R⁴ sont simultanément tous des atomes d'hydrogène; et que R⁵ ne soit pas un groupe phényle, un groupe 4-méthylphényle ou un groupe 4-méthoxyphényle, si R¹, R³ et R⁴ sont simultanément tous des atomes d'hydrogène et si R² est un groupe chloro.

2. Composé de la formule la telle que définie dans la revendication 1, sous l'une ou l'autre de ses formes stéréoisomères ou un mélange de celles-ci selon n'importe quelle proportion, ou un sel pharmaceutiquement acceptable de celui-ci, dans lequel R¹ et R⁴ sont indépendamment l'un de l'autre choisis parmi le groupe constitué d'un atome H, d'halogènes et d'un groupe alkyle en C₁ à C₄, et R² et R³ sont indépendamment l'un de l'autre choisis parmi le groupe constitué d'un atome de H, d'halogènes et d'un groupe alkyle en C₁ à C₄.

3. Composé de la formule Ia telle que définie dans la revendication 1 et/ou 2, sous l'une ou l'autre de ses formes stéréoisomères ou dans un mélange de celles-ci selon n'importe quelle proportion, ou un sel pharmaceutiquement acceptable de celui-ci, dans lequel un ou deux de R¹, R², R³ et R⁴ ne sont pas des atomes d'hydrogène.

4. Composé de la formule la telle que définie dans la revendication 1 et/ou 2, sous l'une ou l'autre de ses formes stéréoisomères ou dans un mélange de celles-ci selon n'importe quelle proportion, ou un sel pharmaceutiquement acceptable de celui-ci, dans lequel R¹, R², R³ et R⁴ sont tous des atomes d'hydrogène.

5. Composé de la formule la telle que définie dans une ou plusieurs des revendications 1 à 4, sous l'une ou l'autre de ses formes stéréoisomères ou dans un mélange de celles-ci selon n'importe quelle proportion, ou un sel pharmaceutiquement acceptable de celui-ci, dans lequel R⁵ est un groupe phényle qui est non substitué ou qui porte un ou plusieurs substituants identiques ou différents choisis parmi le groupe constitué de :
des halogènes ; un groupe -CN ; un groupe NH₂ ; des groupes alkyle en C₁ à C₆, alcényle en C₂ à C₆, alcynyle en C₂ à C₆, alcoxy en C₁ à C₃, alkyle en C₁ à C₄-amino et di (alkyle en C₁ à C₄)amino non substitués et au moins monosubstitués, dont les substituants sont choisis parmi le groupe constitué de l'atome F, des groupes alcoxy en C₁ à C₃, alkyle en C₁ à C₃-mercapto et NH₂ ; 3 un groupe alcanediyle en C₃ à C₅ ; un groupe phényle ; un groupe hétéroaryle ; un groupe alkyle en C₁ à C₂ à substitution phényle ou à substitution hétéroaryle ; un groupe CF₃ ; un groupe OH ; un groupe (alkyle en C₁ à C₄)-COO ; un groupe S(O)ₘ-(C₁à C₄) -alkyle ; un groupe (alkyle en C₁ à C₄)-CONH- ; un groupe (alkyle en C₁ à C₄)-CON(alkyle en C₁ à C₄)-; un groupe (alkyle en C₁ à C₄-alkyl)-CO- ; un groupe phényl-CO- ; un groupe hétéroaryl-CO- ; un groupe CF₃-CO- ; un groupe -OCH₂O- ; un groupe -OCF₂O- ; un groupe -OCH₂CH₂O- ; un groupe -CH₂CH₂O-; un groupe COO(alkyle en C₁ à C₆); un groupe -CONH₂; un groupe -CONH(alkyle en C₁ à C₄); un groupe -CON(di(alkyle en C₁ à C₄)) ; un groupe C(NH)NH₂ ; un groupe -SO₂NH₂ ; un groupe -SO₂NH (alkyle en C₁ à C₄); un groupe -SO₂NH(phényle) ; un groupe -SO₂N(di(alkyle en C₁ à C₄) ; un groupe (alkyle en C₁ à C₄)-SO₂NH- ; un groupe (alkyle en C₁ à C₄)-SO₂N(alkyle en C₁ à C₄)- ; et un résidu d'un hétérocycle aliphatique mononucléaire de cinq chaînons à sept chaînons, saturé ou au moins monoinsaturé, comprenant 1, 2 ou 3 hétéroatomes choisis parmi le groupe constitué des atomes N, O et S, lequel hétérocycle peut être substitué par un ou plusieurs substituants choisis parmi le groupe constitué d'halogènes, des groupes alkyle en C₁ à C₃, alcoxy en C₁ à C₃, OH, oxo et CF₃, où ledit hétérocycle peut éventuellement être condensé en ledit groupe phényle, dans lequel tous les groupes hétéroaryle, phényle, contenant un groupe hétéroaryle et contenant un groupe phényle, lesquels sont éventuellement présents dans lesdits substituants dudit groupe phényle, peuvent être substitués par un ou plusieurs substituants choisis parmi le groupe constitué d'halogènes, des groupes -CN, alkyle en C₁ à C₃, OH, alcoxy en C₁ à C₃ et CF₃.

6. Composé de la formule Ia telle que définie dans une ou plusieurs des revendications 1 à 5, sous l'une ou l'autre de ses formes stéréoisomères ou dans un mélange de celles-ci selon n'importe quelle proportion, ou un sel pharmaceutiquement acceptable de celui-ci, dans lequel R⁵ est un groupe phényle qui est non substitué ou qui porte un ou plusieurs substituants identiques ou différents choisis parmi le groupe constitué de :
un atome F ; un atome Cl ; un atome Br ; un groupe alkyle en C₁ à C₃ ; un groupe alcoxy en C₁ à C₃-méthyle en C₁ à C₃ ; un groupe 2-amino-3,3,3-trifluoropropyl- ; un groupe CF₃ ; un groupe alcanediyle en C₃ à C₅ ; un groupe phényle ; un groupe hétéroaryle ; un groupe benzyle ; un groupe hétéroaryl-méthyl- ; un groupe OH ; un groupe alcoxy en C₁ à C₃ ; un groupe phénoxy ; un groupe trifluorométhoxy ; un groupe 2,2,2-trifluoroéthoxy ; un groupe (alkyle en C₁ à C₄)-COO ; un groupe alkyle en C₁ à C₃-mercapto ; un groupe phénylmercapto ; un groupe alkylsulfonyle en C₁ à C₃; un groupe phénylsulfonyle; un groupe NH₂; un groupe alkyle en C₁ à C₄-amino ; un groupe di (alkyle en C₁ à C₄)amino, un groupe (alkyle en C₁ à C₃)-CONH- ; un groupe (alkyle en C₁-C₃)-SO₂NH- ; un groupe (alkyle en C₁ à C₃)-CO- ; un groupe phényl-CO- ; un groupe -OCH₂O-; un groupe -OCF₂O-; un groupe -CH₂CH₂O-; un groupe COO(C₁-C₄-alkyl); un groupe -CONH₂; un groupe -CONH(alkyle en C₁ à C₄); un groupe -CON (di (alkyle en C₁ à C₄)); un groupe -CN ; un groupe -SO₂NH₂ un groupe -SO₂NH(alkyle en C₁ à C₄); un groupe -SO₂N(di(alkyle en C₁ à C₄)) ; un groupe pyrrolidinyle ; un groupe pipéridinyle ; un groupe morpholinyle et un groupe thiomorpholinyle ; dans lequel tous les groupes hétéroaryle, phényle, contenant un groupe hétéroaryle et contenant un groupe phényle, lesquels sont éventuellement présents dans lesdits substituants dudit groupe phényle, peuvent être substitués par un ou plusieurs substituants choisis parmi le groupe constitué d'halogènes, des groupes -CN, alkyle en C₁ à C₃, OH, alcoxy en C₁ à C₃ et CF₃.

7. Composé de la formule Ia telle que définie dans une ou plusieurs des revendications 1 à 6, sous l'une ou l'autre de ses formes stéréoisomères ou dans un mélange de celles-ci selon n'importe quelle proportion, ou un sel pharmaceutiquement acceptable de celui-ci, pour une utilisation comme médicament.

8. Préparation pharmaceutique, comprenant une dose efficace d'au moins un composé de la formule Ia telle que définie dans une ou plusieurs des revendications 1 à 6, sous l'une ou l'autre de ses formes stéréoisomères ou dans un mélange de celles-ci selon n'importe quelle proportion, et/ou un sel pharmaceutiquement acceptable de celui-ci et un vecteur pharmaceutiquement acceptable.

9. Utilisation d'un composé de la formule Ia telle que définie dans une ou plusieurs des revendications 1 à 6, sous l'une ou l'autre de ses formes stéréoisomères ou dans un mélange de celles-ci selon n'importe quelle proportion, ou un sel pharmaceutiquement acceptable de celui-ci, pour la fabrication d'un médicament pour le traitement de maladies cardiovasculaires, de l'angine de poitrine stable ou instable, de la maladie coronarienne, de l'angor de Prinzmétal, du syndrome coronarien aigu, d'une insuffisance cardiaqùe, d'un infarctus du myocarde, de l'accident vasculaire cérébral, de la thrombose, de la maladie de l'occlusion des artères périphériques, de la dysfonction endothéliale, de l'athérosclérose, de la resténose, des dommages endothéliaux après une ACTP, de l'hypertension, de l'hypertension artérielle essentielle, de l'hypertension artérielle pulmonaire, de l'hypertension artérielle secondaire, de l'hypertension artérielle rénovasculaire, de la glomérulonéphrite chronique, d'une dysfonction érectile, de l'arythmie ventriculaire, du diabète, des complications du diabète, d'une néphropathie, d'une rétinopathie, d'une angiogenèse, de l'asthme bronchique, de l'insuffisance rénale chronique, de la cirrhose du foie, de l'ostéoporose, du fonctionnement limité de la mémoire ou d'une capacité restreinte d'apprentissage, ou pour réduire le risque cardiovasculaire des femmes postménopausées ou des femmes qui prennent des contraceptifs.
